(19) Europäisches Patentamt
European Patent Office
Office européen des brevets

(11) EP 1 806 128 B1

(12) **EUROPÄISCHE PATENTSCHRIFT**

(45) Veröffentlichungstag und Bekanntmachung des Hinweises auf die Patenterteilung:
**02.09.2009 Patentblatt 2009/36**

(51) Int Cl.:
***A61K 8/898*** *(2006.01)* ***A61Q 5/00*** *(2006.01)*

(21) Anmeldenummer: **06025998.3**

(22) Anmeldetag: **15.12.2006**

(54) **Haarpflegemittel**

Hair care product

Produit de soin pour cheveux

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.12.2005 DE 102005062651**

(43) Veröffentlichungstag der Anmeldung:
**11.07.2007 Patentblatt 2007/28**

(73) Patentinhaber: **Henkel AG & Co. KGaA**
**40589 Düsseldorf (DE)**

(72) Erfinder:
- **Reichert, Anja**
  **40629 Düsseldorf (DE)**
- **Seiler, Martina**
  **47228 Duisburg (DE)**
- **Goutsis, Konstantin**
  **41812 Erkelenz (DE)**

(56) Entgegenhaltungen:
**EP-A1- 0 819 422**
**WO-A-20/07000223**
**DE-A1- 10 304 923**
**JP-A- 10 114 628**
**US-A- 5 376 146**

- **NRC NORDMANN ET AL: "Starke Haarfarben dank Baysilone TP 3911" INTERNET CITATION, [Online] 17. Februar 2005 (2005-02-17), XP002394906 Gefunden im Internet: URL:http: //web.archive.org/web/20050217172 802/www.nrc.de/da-presse-0081.html> [gefunden am 2006-08-16]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 806 128 B1

## Beschreibung

Die vorliegende Erfindung betrifft Mittel zur Konditionierung keratinhaltiger Fasern, die eine spezielle Pflegemittelkombination aus mindestens einem speziellen amphoteren Tensid, mindestens einem amphoteren und/oder kationischen Polymer und mindestens einer Polyammonium-Polysiloxan-Verbindung enthalten. Ferner wird Verfahren zur Haarbehandlung mit diesem Mittel beschrieben.

Nicht zuletzt durch die starke Beanspruchung der Haare, beispielsweise durch Umweltbelastungen, das Färben oder Dauerwellen als auch durch die Reinigung der Haare mit Shampoos, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Nass- und Trockenkämmbarkeit des Haares, der Halt und die Fülle des Haares optimiert sein oder die Haare vor erhöhtem Spliss geschützt sein.

Es ist daher seit langem üblich, die Haare einer speziellen Behandlung zu unterziehen. Dabei werden, üblicherweise in Form einer Spülung, die Haare mit speziellen Pflegestoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splissrate verringert.

Weiterhin wurden in jüngster Zeit so genannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung oder Färbung der Haare, zusätzlich Pflegestoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Pflegestoffe sowohl für separate Nachbehandlungsmittel als auch für Kombinationspräparate wirken im Allgemeinen bevorzugt an der Haaroberfläche. So sind Pflegestoffe bekannt, welche dem Haar Glanz, Halt, Fülle, bessere Nass- oder Trockenkämmbarkeiten verleihen oder dem Spliss vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflusst werden kann.

Es besteht daher weiterhin ein Bedarf nach Pflegestoffen bzw. Pflegestoffkombinationen für kosmetische Mittel mit guten pflegenden Eigenschaften und guter biologischer Abbaubarkeit.

Pflegestoffe können in die verschiedensten kosmetischen Träger eingearbeitet werden. Als kosmetische Träger dienen dabei Lotionen, Shampoos oder O/W- bzw. W/O-Emulsionen. Insbesondere die Form eines klaren Gels stellt einen speziellen, reizvollen kosmetischen Träger für Pflegestoffe dar.

Wenn Konditioniermittel in Form eines klaren Gels formuliert werden sollen, stellt sich für den Fachmann die Aufgabe, die Pflegestoffe des Konditioniermittels so auszuwählen, dass sich der Pflegestoff dauerhaft in den gelförmigen kosmetischen Träger einarbeiten lässt. Das resultierende Gel sollte stets stabil und klar bleiben, d.h. z.B. bei längerer Lagerung, insbesondere bei Temperaturen von -20°C bis 60°C, sollte keine Phasentrennung oder Trübung auftreten. Die Pflegeleistung darf bei Berücksichtigung der vorherigen Gesichtspunkte nicht beeinträchtigt werden.

Die Konditioniermittel sollen sich ebenso hervorragend zur Formulierung von Kombinationspräparaten, insbesondere Kombinationspräparate auf Basis oxidativer Färbemittel, eignen.

Für das Färben von Keratinfasern, insbesondere von menschlichen Haaren, spielen die so genannten Oxidationsfärbemittel wegen ihrer intensiven Farben und guten Echtheitseigenschaften eine bevorzugte Rolle. Solche Färbemittel enthalten Oxidationsfarbstoffvorprodukte, so genannte Entwicklerkomponenten und Kupplerkomponenten.

Die Entwicklerkomponenten tragen, wenn sie als Aromaten vorliegen, mindestens eine gegebenenfalls substituierte Aminogruppe und mindestens eine dazu in ortho- oder para-Position befindliche Amino- oder Hydroxygruppe, welche gegebenenfalls ebenso substituiert sein kann.

Spezielle Vertreter der Entwicklerkomponenten sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diamino-propan-2-ol.

Als Kupplerkomponenten werden in der Regel m-Phenylendiaminderivate, Naphthole, Resorcin und Resorcinderivate, Pyridine, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, 2-Amino-3-hydroxypyridin, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol. Die Kupplerkomponenten tragen, wenn sie als aromatische Verbindungen vorliegen, keine zueinander ortho- oder para-ständigen Amino- bzw. Hydroxygruppen, welche gegebenenfalls substituiert sein können.

Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Meist wird ein Oxidationsmittel wie Wasserstoffperoxid direkt vor der Anwendung den Oxidationsfarbstoffvorprodukten zugemischt.

Die oxidativen Färbemittel werden daher oft als Verkaufseinheit (Kit) mit mindestens zwei getrennt konfektionierten Komponenten angeboten:

Die Oxidationsfarbstoffvorprodukte sind als erste Komponente in einer meist auf einen basischen pH-Wert eingestellten Färbecreme oder einem Färbegel enthalten. Färbegele sind nicht nur ästhetischer und daher ansprechender für den Verbraucher. Zusätzlich bieten Gele aufgrund ihrer Strukturviskosität einen Vorteil bei der Anwendung am Haar. Besonders wichtig ist daher, dass nach dem Auftragen auf die Fasern die Strukturviskosität des erfindungsgemäßen Konditioniermittels erhalten bleibt und letzteres sich zwar leicht auf dem Haar verteilen lässt, nicht aber vom Haar abläuft.

Die Oxidationsmittel sind in diesem Falle in einer gesondert verpackten Oxidationsmittelzubereitung, meist im Form einer Lotion, Emulsion oder Dispersion konfektioniert, als zweite Komponente in dem Kit enthalten. Die Oxidationsmittelzubereitung besitzt üblicherweise einen sauren pH-Wert.

Die Anwendungsmischung aus der ersten und zweiten Komponente besitzt üblicherweise einen basischen pH-Wert. Die Pflegestoffe müssen folglich auch bei basischem pH-Wert eine angemessene Pflegeleistung erbringen. Erstrebenswerterweise liefern die Pflegestoffe auch in Gegenwart von Oxidationsmitteln, wie z.B. Wasserstoffperoxid, und/oder hohen Elektrolytkonzentrationen ein hervorragendes Pflegeergebnis.

Gute oxidative Färbemittel bilden bei der oxidativen Kupplung der Vorprodukte die gewünschten Farbnuancen in ausreichender Intensität und Echtheit aus. Die Oxidationsfarbstoffvorprodukte sollen ferner ein gutes Aufziehvermögen auf die Faser besitzen, wobei insbesondere bei menschlichen Haaren keine merklichen Unterschiede zwischen strapaziertem und frisch nachgewachsenem Haar bestehen sollen (Egalisiervermögen). Die erzielten Färbungen sollen beständig sein gegen Licht, Wärme, Schweiß, Reibung und den Einfluss chemischer Reduktionsmittel, z.B. Dauerwellenflüssigkeiten. Schließlich sollen die Mittel - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Zusätzlich zu hervorragenden färberischen Eigenschaften stellt der Verbraucher an die modernen oxidativen Färbemittel den Anspruch der Haarpflege.

Wie in Färbeverfahren mit herkömmlichen oxidativen Haarfärbemitteln üblich, wird nach dem Färbevorgang ein so genanntes Konditioniermittel im Rahmen einer Nachbehandlung angewendet. Diese Konditioniermittel weisen einen sauren bis neutralen pH-Wert auf und sind oft in kommerziell angebotenen oxidativen Färbemittel-Kits enthalten. Diese Konditioniermittel dienen der Avivage und sollen in manchen Fällen den Farberhalt verbessern.

Seit geraumer Zeit sind oxidative Haarfärbemittel bekannt, welche neben der Haarfärbung gleichzeitig avivierend wirken. Die Haarpflege (Avivage) wird bei diesen 2-in-1-Färbemitteln meist durch Zusatz von bekannten Pflegestoffen zum oxidativen Haarfärbemittel erreicht. Im handelsüblichen Haarfärbe-Kit werden die Pflegestoffe entweder der oxidationsmittelhaltigen Komponente, separat konfektioniert bzw. der Färbecreme zugesetzt, je nach dem, ob der betroffene Pflegestoff lagerstabil in eine Oxidationsmittelzubereitung eingearbeitet werden kann oder nicht.

Meist erfolgt auch bei Anwendung der 2-in-1-Färbemittel nach der oxidativen Färbung eine Nachbehandlung mit einem sauer eingestellten Konditioniermittel.

Ein Problem der 2-in-1-Färbemittel ist es, dass die darin enthaltenen Pflegestoffe die Farbaufnahme des Haars beeinträchtigen können. Dies kann insbesondere bei polymeren Pflegestoffen der Fall sein. Darüber hinaus können möglicherweise die zugesetzten Pflegestoffe die Eigenschaften der Färbungen negativ beeinflussen.

Die Druckschrift US-A-4 507 280 betrifft Haarkonditioniermittel, welche neben 0.1 bis 10 % eines kationischen Polymers, 0.2 bis 20 % eines amphoteren Tensids in einem definierten Mengenverhältnis zueinander enthalten. Die Konditioniermittel enthalten nicht die erfindungsgemäße Wirkstoffkombination.

Aus der Druckschrift EP-B1-640 335 sind zwei Komponenten Färbemittel bekannt, welche in einer ersten Komponente eine alkalische wässrige Lösung von Entwickler und Kupplerkomponenten in Kombination mit einem kationischen Polymer, einem anionischen und/oder amphoteren Tensid und mindestens 70% Wasser enthalten. In einer zweiten, auf einen sauren pH-Wert eingestellten Komponente sind mindestens ein Oxidationsmittel und ein dispergiertes anionisches Polymer enthalten. Nach der Mischung der Komponenten bildet sich ein Gel mit einem pH-Wert von größer pH 7. Dabei löst sich das anionische Polymer auf, das kationische Polymer jedoch bildet einen unlöslichen Niederschlag. Die in dieser Druckschrift beschriebenen Mittel enthalten nicht die erfindungsgemäße Wirkstoffkombination.

Die Verwendung von Polyammonium-Polysiloxan-Verbindungen wird beispielsweise in der WO 2004/069137 sowie der WO 02/10257 beschrieben. Die in diesen Druckschriften erwähnten Mittel enthalten ebenso nicht die erfindungsgemäße Wirkstoffkombination.

DE 103 04 923 A1 (GE BAYER SILICONES GMBH & CO [DE]) 26. August 2004 (2004-08-26) offenbart (Ansprüche 16-19) für die Haarkonditionierung geeignete Zusammensetzungen, enthaltend:

- ein kationisches und/oder amphoteres Polymer und
- eine Polyammonium-Polysiloxan-Verbindung.

Aufgabe der vorliegenden Erfindung ist es, neue Konditioniermittel für keratinhaltige Fasern bereitzustellen, die lagerstabil sind und zusätzlich verbesserte haarkonditionierende Eigenschaften, insbesondere bei einer oder mehrerer der eingangs beschriebenen Bedingungen, besitzen. Die Konditioniermittel sollen bevorzugt als pflegend wirkende Oxidationsfärbemittel ausgeführt werden und müssen in diesem Falle zusätzlich Färbungen mit guten Echtheitseigenschaften, insbesondere in Bezug auf die Wasch-, Reib-, Licht-, Kaltwell- und Schweißechtheit, in einem breiten Farbspektrum liefern und in dermatologischer Hinsicht unproblematisch sein.

Ein erster Gegenstand der Erfindung sind wasserhaltige Mittel zur Konditionierung keratinhaltiger Fasern, insbesondere menschlicher Haare, enthaltend eine Kombination aus

- mindestens einem amphoteren Tensid gemäß Formel (I) und/oder dessen physiologisch verträglichem Salz,

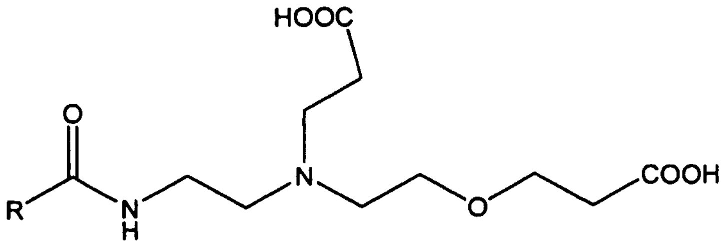

(I)

worin R eine lineare oder verzweigte, gesättigte oder ungesättigte ($C_{10}$ - $C_{22}$)-Alkylgruppe bedeutet,
- mindestens einem amphoteren und/oder kationischen Polymer
und
- mindestens einer Polyammonium-Polysiloxan-Verbindung.

Das erfindungsgemäße Mittel bewirkt eine hervorragende Avivage, insbesondere eine verbesserte Nasskämmbarkeit. Wenn die Konditioniermittel als Gel vorliegen, wird ein klares und lagerstabiles Gel erhalten.

Unter keratinhaltigen Fasern sind erfindungsgemäß Pelze, Wolle, Federn und insbesondere menschliche Haare zu verstehen. Obwohl sich die erfindungsgemäßen Konditioniermittel in erster Linie zur Konditionierung von keratinhaltigen Fasern eignen, steht prinzipiell einer Verwendung auch auf anderen Gebieten nichts entgegen.

Ein erfindungsgemäßes wasserhaltiges Mittel enthält bevorzugt Wasser in einer Menge von 50 bis 99 Gew.-%, besonders bevorzugt von 60 bis 85 Gew.-%, jeweils bezogen auf das Gewicht des Mittels.

Die erfindungsgemäßen Mittel enthalten mindestens eine Polyammonium-Polysiloxan Verbindung, die wie im Folgenden beschrieben, aufgebaut ist. Die Polyammonium-Polysiloxan Verbindungen enthalten:

a1) mindestens eine Polyalkylenoxid-Struktureinheit der allgemeinen Formeln:

- A-E-, -E-A-, -A-E-A'- und/oder -A'-E-A- , worin:

A steht für eine der Gruppen: $-CH_2C(O)O-$, $-CH_2CH_2C(O)O-$, $-CH_2CH_2CH_2C(O)O-$, $-OC(O)CH_2-$, $-OC(O)CH_2CH_2-$ und/oder $-OC(O)CH_2CH_2CH_2-$,

A' bedeutet: $-CH_2C(O)-$, $-CH_2CH_2C(O)-$, $-CH_2CH_2CH_2C(O)-$, $-C(O)CH_2-$, $-C(O)CH_2CH_2-$ und/oder $-C(O)CH_2CH_2CH_2-$ und

E steht für eine Polyalkylenoxidgruppe der allgemeinen Formeln:

$-[CH_2CH_2O]_q-[CH_2CH(CH_3)O]_r-$ und/oder $-[OCH(CH_3)CH_2]r,-[OCH_2CH_2]_q-$, mit q = 1 bis 200 und r = 0 bis 200, wobei das endständige Sauerstoffatom der Gruppe A an die endständige $-CH_2$-Gruppe der Gruppe E, und das endständige Carbonylkohlenstoffatom der Gruppe A' an das endständige Sauerstoffatom Gruppe E jeweils unter Ausbildung von Estergruppen binden, und/oder mindestens eine endständige Polyalkylenoxid-Struktureinheit der Formel $-A-E-R^2$, worin A und E die oben genannte

Bedeutung aufweisen, und $R^2$ steht für H, geradkettiger, cyclischer oder verzweigter $C_1$ - $C_{20}$ - Kohlenwasserstoffrest, der durch -O-, oder - C(O)unterbrochen und mit -OH substituiert und acetylenisch, olefinisch oder aromatisch sein kann,

a2) mindestens einen zweiwertigen oder dreiwertigen organischen Rest, der mindestens eine Ammoniumgruppe enthält,

a3) mindestens eine Polysiloxan- Struktureinheit der allgemeinen Formel:

-K-S-K-,

worin S steht für $-Si(R^1)_2-O[-Si(R^1)_2-O]_n-Si(R^1)_2-$ und
worin $R^1$ steht für $C_1$-$C_{22}$-Alkyl, $C_1$-$C_{22}$-Fluoralkyl oder Aryl, n steht für 0 bis 1000, und wenn mehrere Gruppen S in der Polysiloxanverbindung vorliegen, diese gleich oder verschieden sein können,
worin K ein zweiwertiger oder dreiwertiger geradkettiger, cyclischer oder verzweigter $C_2$-$C_{40}$-Kohlenwasserstoffrest, der durch -O-, -N -, $-NR^1-$, -C(O)-, -C(S)-, $-N^+(R^3)-$ und $-N^+(R^1)(R^3)-$ unterbrochen und mit -OH substituiert sein kann,
worin $R^1$ wie oben definiert ist, oder gegebenenfalls eine Bindung zu einem zweiwertigen Rest $R^3$ darstellt, und
worin $R^3$ einen einwertigen oder zweiwertigen geradkettigen, cyclischen oder verzweigten $C_1$-$C_{20}$-Kohienwasserstoffrest, der durch -O-, - NH-, -C(O)-, -C(S)- unterbrochen und mit -OH substituiert sein kann, oder $-A-E-R^2$ darstellt, worin A, E und $R^2$ wie oben definiert ist,
wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt,

a4) einen organischen oder anorganischen Säurerest zur Neutralisation der aus der(n) Ammoniumgruppe(n) resultierenden Ladungen.

Die erfindungsgemäßen Polysiloxanverbindungen sind dadurch gekennzeichnet, dass sie die vorstehend definierten Komponenten a1) bis a4) aufweisen. Die Polysiloxanverbindungen werden dabei durch Bindung der genannten Struktureinheiten bzw. Reste a1) bis a3) aneinander gebildet. Die Komponente a4) dient der Neutralisation der aus der Komponente a2) resultierenden positiven Ladungen.
Die erfindungsgemäßen Polysiloxanverbindungen können Oligomere oder polymere Verbindungen sein. Oligomere Verbindungen schließen dabei auch den unten beschriebenen Fall ein, worin die Polysiloxanverbindung lediglich eine Wiederholungseinheit aufweist.
Polymere erfindungsgemäße Polysiloxanverbindungen entstehen dabei naturgemäß durch alternierende Verknüpfung von zweiwertigen Resten.
Im Falle der polymeren erfindungsgemäßen Polysiloxanverbindungen resultieren die endständigen Atomgruppierungen aus den endständigen Atomgruppierungen der eingesetzten Ausgangsmaterialien. Dies ist dem Fachmann an sich bekannt.
In einer bevorzugten Ausführungsform sind die polymeren erfindungsgemäßen Polysiloxanverbindungen lineare Polyammonium-Polysiloxanverbindungen, die sich aus den Struktur-Komponenten a1) bis a3) zusammensetzen. So können die linearen polymeren erfindungsgemäßen Polysiloxanverbindungen, insbesondere deren aus den Wiederholungseinheiten gebildete lineare polymere Hauptkette, durch alternierende Aneinanderreihung von Polyalkylenoxid-Struktureinheiten a1), organischen Resten, die mindestens eine, vorzugsweise quartäre Ammoniumgruppe enthalten a2) und Polysiloxan- Struktureinheiten a3) aufgebaut werden. Das heißt, die darüber hinaus gegebenenfalls in den Strukturkomponenten vorhandenen freien Valenzen (wie sie bei dreiwertigen Resten als Komponente a2) oder bei dreiwertigen Resten K auftreten können) dienen bevorzugt nicht dem Aufbau polymerer Seitenketten bzw. polymerer Verzweigungen.
In einer weiteren Ausführungsform kann die Hauptkette der linearen polymeren erfindungsgemäßen Polysiloxanverbindungen von den organischen Resten, die mindestens eine Ammoniumgruppe enthalten a2) und den Polysiloxan - Struktureinheiten a3) aufgebaut werden, und die Polyalkylenoxid- Struktureinheiten a1) binden als Seitenketten an den dreiwertigen organischen Ammoniumgruppenrest.
So können beispielsweise folgende Aufbauten resultieren:

- (Polyalkylenoxidstruktureinheit-Polysiloxanstruktureinheit- Polyalkylenoxidstruktureinheit - bevorzugt quartärer Ammnoniumgruppenrest)$_x$- -(Polysiloxanstruktureinheit - bevorzugt quartärer Ammoniumgruppenrest)$_x$-Polyalkylenoxidstruktureinheit)$_x$-
- (Polysiloxanstruktureinheit- bevorzugt quartärer Ammoniumgruppenrest)$_x$-Polyalkylenoxidstruktureinheit

Je nach molarem Verhältnis der monomeren Ausgangsverbindungen können erfindungsgemäße Polysiloxanverbindungen resultieren, die lediglich eine Wiederholungseinheit aufweisen. Dies ist dem Fachmann an sich bekannt. Dieser Fall führt beispielsweise zu erfindungsgemäßen Polysiloxanverbindungen des Aufbaus:

(endständige Polyalkylenoxidstruktureinheit-quartärer Ammoniumgruppenrest-Polysiloxanstruktureinheit-quartärer Ammoniumgruppenrest- endständige Polyalkylenoxidstruktureinheit).

Die erfindungsgemäße Polysiloxanverbindungen bestehen bevorzugt im wesentlichen aus den Komponenten a1) bis a4), wobei die polymeren erfindungsgemäßen Polysiloxanverbindungen naturgemäß die aus der Umsetzung der monomeren Ausgangsmaterialien resultierenden terminalen Gruppen aufweisen. Es können aber auch monofunktionelle Kettenabbruchsmittel eingesetzt werden.

Bei den Polyalkylenoxid-Struktureinheiten a) kann es sich um zweiwertige Reste der allgemeinen Formeln:

-A-E-, -E-A-, -A-E-A'- und/oder -A'-E-A- handeln.

Der Rest A bedeutet dabei:

$-CH_2C(O)O-$, $-CH_2CH_2C(O)O-$, $-CH_2CH_2CH_2C(O)O-$, $-OC(O)CH_2-$, $-OC(O)CH_2CH_2-$ und/oder $-OC(O)CH_2CH_2CH_2-$

Der Rest A' bedeutet dabei:

$-CH_2C(O)-$, $-CH_2CH_2C(O)-$, $-CH_2CH_2CH_2C(O)-$, $-C(O)CH_2-$, $-C(O)CH_2CH_2-$ und/oder $-C(O)CH_2CH_2CH_2-$.

Die Polyalkylenoxidgruppe E der allgemeinen Formeln: $-[CH_2CH_2O]_q-[CH_2CH(CH_3)O]_r-$ und/oder $-[OCH(CH_3)CH_2]_r-[OCH_2CH_2]_q$ mit q = 1 oder 2 bis 200 und r = 0 bis 200, schließen dabei alle möglichen Ethylenoxid/Propylenoxid-Gruppierungen ein. So kann es sich um statistische Ethylenoxid/Propylenoxid-Copolymergruppen oder Ethylenoxid/Propylenoxid-Block Copolymergruppen mit beliebiger Anordnung von einem oder mehreren Ethylenoxid-, oder Propylenoxid-Blöcken handeln.

Die Anbindung der Reste A bzw. A' an die Gruppe E erfolgt dabei so, dass das endständige Sauerstoffatom der Gruppe A an die endständige $-CH_2-$ Gruppe der Gruppe E, und das endständige Carbonyl-Kohlenstoffatom der Gruppe A' an das endständige Sauerstoffatom der Gruppe E jeweils unter Ausbildung von Estergruppen binden.

Bei den Polyalkylenoxid-Struktureinheiten a1) kann es sich weiterhin um einwertige, d.h. endständige Polyalkylenoxid-Struktureinheit der Formel - $A-E-R^2$ handeln, worin A und E die oben genannte Bedeutung aufweisen, und $R^2$ für H, geradkettiger, cyclischer oder verzweigter $C_1$-$C_{20}$-Kohlenwasserstoffrest steht, der durch -O-, oder -C(O)- unterbrochen und mit -OH substituiert und acetylenisch, olefinisch oder aromatisch sein kann.

Die Komponente a2) aus der sich die erfindungsgemäßen Polysiloxanverbindungen zusammensetzen, ist mindestens ein zweiwertiger oder dreiwertiger organischer Rest, der mindestens eine Ammoniumgruppe enthält. Die Bindung des Restes an die übrigen Komponenten der erfindungsgemäßen Polysiloxanverbindungen erfolgt bevorzugt über das Stickstoffatom einer oder mehrerer Ammoniumgruppen in dem organischen Rest. Der Begriff "zweiwertig" bzw. "dreiwertig" bedeutet, dass der organische Ammonium-Rest zur Ausbildung von Bindungen insbesondere zu den übrigen Komponenten der erfindungsgemäßen Polysiloxanverbindungen zwei oder drei freie Valenzen aufweist. Der Ammoniumrest wird zweckmäßig durch eine $NH_4^+$-Gruppe dargestellt, in der mindestens zwei Wasserstoffatome durch organische Gruppen substituiert sind. Vorzugsweise handelt es sich um eine sekundäre oder quartäre, besonders bevorzugt um eine quartäre Ammoniumgruppe. Eine quartäre Ammoniumgruppe ist nach allgemeiner Definition (s. z.B. Römpp-Chemie-Lexikon) eine Gruppe bei der alle vier Wasserstoffatome einer $NH_4^+$-Gruppe durch organische Reste ersetzt sind.

Die Komponente a2) der erfindungsgemäßen Polysiloxanverbindungen ist mindestens eine Polysiloxan-Struktureinheit der allgemeinen Formel:

-K-S-K-,

S ist darin eine Polysiloxangruppe der allgemeinen Formel $-Si(R^1)_2-O[-Si(R^1)_2-O]_n-Si(R^1)_2-$,
worin R1 bedeutet $C_1$-$C_{22}$-Alkyl, $C_1$-$C_{22}$-Fluoralkyl oder Aryl, vorzugsweise Phenyl, n = 0 bis 1000, und wenn mehrere Gruppen S in der Polysiloxanverbindung vorliegen, diese gleich oder verschieden sein können.

$R^1$ ist bevorzugt $C_1$-$C_{18}$-Alkyl, $C_1$-$C_{18}$-Fluoralkyl und Aryl. Weiterhin ist R1 bevorzugt $C_1$-$C_{18}$-Alkyl, $C_1$-$C_6$-Fluoralkyl und Aryl. Weiterhin ist $R^1$ bevorzugt $C_1$-$C_6$-Alkyl, $C_1$ - $C_6$-Fluoralkyl, bevorzugter $C_1$-$C_4$-Fluoralkyl, und Phenyl. Noch bevorzugter ist $R^1$ Methyl, Ethyl, Trifluorpropyl und Phenyl.

Der Begriff "$C_1$-$C_{22}$-Alkyl" bedeutet im Rahmen der vorliegenden Erfindung, dass die aliphatische Kohlenstoffwasserstoffgruppen 1 bis 22 Kohlenstoffatome besitzen, die geradkettig oder verzweigt sein können. Beispielhaft seien Methyl, Ethyl, Propyl, n-Butyl, Pentyl, Hexyl, Heptyl, Nonyl, Decyl, Undecyl, iso-Propyl, Neopentyl, und 1,2,3 Trimethylhexyl aufgeführt.

Der Begriff "$C_1$-$C_{22}$-Fluoralkyl" bedeutet im Rahmen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2-Triflourbutyl aufgeführt.

Der Begriff "Aryl" bedeutet im Rahmen der vorliegenden Erfindung unsubstituierte oder ein oder mehrfach mit OH, F, Cl, $CF_3$ $C_1$-$C_6$-Alkyl, $C_1$-$C_6$-Alkoxy, $C_3$-$C_7$- Cycloalkyl $C_2$-$C_6$-Alkenyl oder Phenyl substituiertes Phenyl. Der Ausdruck kann gegebenenfalls auch Naphthyl bedeuten.

K stellt einen zweiwertigen oder dreiwertigen geradkettigen, cyclischen oder verzweigten $C_2$-$C_{40}$-Kohlenwasserstoffrest dar, der durch -O-, - NH-, -N-, C(O)-,-C(S)-, -$N^+(R^3)$-, -$NR^1$-, und -$N^+(R^1)(R^3)$- unterbrochen und mit -OH substituiert sein kann.

"Unterbrochen" bedeutet dabei, dass im Falle der zweiwertigen Reste eine - $CH_2$-Gruppierung im Falle der dreiwertigen Reste eine -CH- Gruppierung des Kohlenwasserstoffrestes durch die genannten Gruppen ersetzt sind. Dies gilt auch für den übrigen Teil der Beschreibung, wenn diese Bezeichnung verwendet wird.

Die Gruppe K bindet über ein Kohlenstoffatom an das Siliziumatom der Gruppe S.

Die Gruppe K kann, wie oben zu sehen, ebenfalls bevorzugt quartäre Ammoniumgruppen aufweisen, so dass Ammoniumgruppen zusätzlich zu den Ammoniumgruppen in der genannten Komponente a2) in den erfindungsgemäßen Polysiloxanverbindungen resultieren.

Die erfindungsgemäßen Polysiloxanverbindungen können, wie zum Beispiel in dem Rest K, Aminogruppen aufweisen. Die Umsetzung der erfindungsgemäßen Polysiloxanverbindungen mit Säuren führt zu deren Protonierung. Solche protonierte Aminogruppen aufweisende Polysiloxanverbindungen sind im Umfang der vorliegenden Erfindung enthalten.

Die Bindung der Komponente a3), der Polysiloxan-Struktureinheit -K-S-K-, zu den übrigen Aufbaukomponenten über den Rest K erfolgt bevorzugt nicht über ein Stickstoffatom des Restes K.

$R^1$ ist wie oben definiert oder stellt gegebenenfalls eine Bindung zu einem zweiwertigen Rest $R^3$ dar, so dass ein Cyclus resultiert.

$R^3$ stellt einen einwertigen oder zweiwertigen geradkettigen, cyclischen oder verzweigten $C_1$-$C_{20}$-Kohlenwasserstoffrest, der durch -O-, -NH-, -C(O)-, -C(S)- unterbrochen und mit -OH substituiert sein kann, oder -A-E-$R^2$ dar, worin A, E und $R^2$ wie oben definiert ist.

Die Reste K können gleich oder verschieden voneinander sein, und im Falle, dass K einen dreiwertigen Rest darstellt, erfolgt die Absättigung der dritten Valenz über eine Bindung an den - vorstehend genannten organischen Rest, der mindestens eine Ammoniumgruppe enthält.

Die erfindungsgemäßen Polysiloxanverbindungen enthalten weiterhin die Komponente a4), mindestens einen organischen oder anorganischen anionischen Säurerest zur Neutralisation der aus der(n) Ammoniumgruppe(n) resultierenden Ladungen. Organische oder anorganische Säurereste sind Reste, die formal aus der Abspaltung von eines oder mehrerer Protonen aus organischen oder anorganischen Säuren resultieren und schließen beispielsweise ein Halogenide, wie Fluorid, Chlorid, Bromid, Sulfate, Nitrate, Phosphate, Carboxylate, wie Formiat, Acetat, Propionat etc., Sulfonate, Sulfate, Polyethercarboxylate und Polyethersulfate etc. Bevorzugt ist Chlorid. Die organischen oder anorganischen anionischen Säurereste als Komponente a4) der erfindungsgemäßen Polysiloxanverbindungen können gleich oder verschieden voneinander sein. So resultieren aus der Umsetzung der Amine mit Alkylhalogeniden bevorzugt Halogenidionen, während zum Beispiel Carboxylate aus den Carbonsäuren, die bei der Umsetzung von Bisepoxiden mit Aminen zugesetzt werden können, resultieren.

In einer bevorzugten Ausführungsform der erfindungsgemäßen Polysiloxanverbindungen stellt K einen zweiwertigen oder dreiwertigen geradkettigen, cyclischen oder verzweigten $C_2$-$C_{40}$-Kohlenwasserstoffrest dar, der durch -O-, -NH-, -N-, -$NR^1$-, -C(O)-, -C(S)- unterbrochen und mit -OH substituiert sein kann, worin $R^1$ wie oben definiert ist, -und wobei die Reste K gleich oder verschieden voneinander sein können.

Der zuvor genannte organische Rest, der mindestens eine, bevorzugt quartäre Ammoniumgruppe enthält, ist bevorzugt ein Rest der allgemeinen Formel:

$$-N^1-F-N^1-,$$

worin N' eine quartäre Ammoniumgruppe der allgemeinen Formel -$(R^4)N^+(R^5)$- ist, worin $R^4$ einen einwertigen oder zweiwertigen geradkettigen, cyclischen oder verzweigten $C_1$-$C_{20}$-Kohlenwasserstoffrest darstellt, der durch -O-, -NH-, -C(O)-, -C(S)- unterbrochen und mit -OH substituiert sein kann, und $R^5$ ist ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest $R^4$ oder einem vierwertigen Rest F, und die

Reste $R^4$ und $R^5$ innerhalb der Gruppe $-N^1-F-N^1-$ sowie in der Polysiloxanverbindung gleich oder verschieden voneinander sein können,

F ist ein zweiwertiger oder vierwertiger geradkettiger, cyclischer oder verzweigter $C_2$ -$C_{30}$-Kohlenwasserstoffrest, der durch -O-, -NH-, -N-, - C(O)-, -C(S)-, eine Siloxankette S, wobei für S die oben genannten Bezüge gelten, unterbrochen und mit -OH substituiert sein kann.

Bezüglich weiterer Einzelheiten der Definitionen der quartären Ammoniumgruppe der Formel $-N^1-F-N^1-$ (bevorzugte Ausführungsformen etc.) sei auf die Erläuterungen der ersten Ausführungsform der vorliegenden Erfindung zur Komponente a, den Polyammonium-Polysiloxan Verbindungen, in der diese Gruppe realisiert ist, verwiesen, und die auch in diesem allgemeineren Kontext Gültigkeit besitzen.

Der zuvor genannte organische Rest, der mindestens eine, bevorzugt quartäre Ammoniumgruppe enthält, kann weiterhin bevorzugt ein Rest der allgemeinen Formel

$-(R^6)N^+(R^7)-$ sein,

worin $R^6$ ein einwertiger oder zweiwertiger geradkettiger, cyclischer oder verzweigter $C_1$-$C_{30}$-Kohlenwasserstoffrest ist, der durch -O-, -NH-, -C(O)-, -C(S)- unterbrochen und mit -OH substituiert sein kann, oder $R^6$ stellt eine Einfachbindung zu einem dreiwertigen Rest K dar.

$R^7$ ist ein einwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann, oder $-A-E-R^2$, worin $-A-E-R^2$ die oben genannte Bedeutung aufweist, oder eine Einfachbindung zu einem zweiwertigen Rest $R^6$ oder zu einem dreiwertigen Rest K. Die Reste $R^6$ und $R^7$ können gleich oder verschieden voneinander sein.

Bezüglich weiterer Einzelheiten der Definitionen der quartären Ammoniumgruppe der Formel $-(R^6)N^+(R^7)-$ (bevorzugte Ausführungsformen) sei auf die Erläuterungen der zweiten, dritten und vierten Ausführungsform zu den erfindungsgemäß bevorzugten Polyammonium-Polysiloxan Verbindungen verwiesen, in der diese Gruppe realisiert ist, und die auch in diesem allgemeineren Kontext Gültigkeit besitzen.

Der zuvor genannte organische Rest, der mindestens eine Ammoniumgruppe enthält, kann weiterhin bevorzugt ein Rest der allgemeinen Formel:

$-N^5-F^1-N^5-$ sein,
worin $N^5$ eine Ammoniumgruppe der allgemeinen Formel
$-(R^{23})N^+(R^{24})-$ ist, worin
$R^{23}$ Wasserstoff, ein einwertiger oder zweiwertiger geradkettiger, cyclischer oder verzweigter $C_1$-$C_{20}$- Kohlenwasserstoffrest darstellt, der durch -O-, -NH-, -C(O)-, -C(S)- unterbrochen und mit -OH substituiert sein kann,
$R^{24}$ Wasserstoff, ein einwertiger geradkettiger, cyclischer oder verzweigter $C_1$- $C_{20}$-Kohlenwasserstoffrest darstellt, der durch -O-, -NH-, -C(O)-, -C(S) unterbrochen und mit -OH substituiert sein kann, oder eine Einfachbindung zu einem zweiwertigen Rest $R^{23}$ darstellt, und die Reste $R^{23}$ Und $R^{24}$ innerhalb der Gruppe $-N^5-F^1-N^5-$sowie in der Polysiloxanverbindung gleich oder verschieden voneinander sein können,
$F^1$ bedeutet ein zweiwertiger geradkettiger, cyclischer oder verzweigter -N Kohlenwasserstoffrest darstellt, der durch -O-, -NH-, -C(O)-, -N-, -C(S)- oder durch eine Gruppe -E- unterbrochen sein kann,
und worin eine Mehrzahl der Gruppen $N^5$ und $F^1$ jeweils gleich oder verschieden voneinander sein können.

Bezüglich weiterer Einzelheiten der Definitionen der Ammoniumgruppe der Formel $-N^5-F^1-N^5-$ (bevorzugte Ausführungsformen) sei auf die Erläuterungen der fünften Ausführungsform zur Komponente a, den Polyammonium-Polysiloxan Verbindungen der vorliegenden Erfindung verwiesen, in der diese Gruppe beispielhaft realisiert ist, und die auch in diesem allgemeineren Kontext Gültigkeit besitzen.

Im Folgenden werden die erfindungsgemäß bevorzugten Polyammonium-Polysiloxan-Verbindungen anhand von fünf bevorzugten Ausführungsformen dieser Verbindungen näher beschrieben.

Eine besondere Ausführungsform der Polyammonium-Polysiloxan Verbindungen (die im folgenden als erste Ausführungsform bezeichnet wird), worin der zuvor genannte organische Rest, der mindestens eine, bevorzugt quartäre Ammoniumgruppe enthält, als Komponente a2) der erfindungsgemäßen Polysiloxanverbindungen einen Rest der allgemeinen Formel:

$-N^1-F-N^1-$

darstellt, wird durch die Polysiloxan-Verbindungen der folgenden allgemeinen Formel (I) dargestellt:

$-[B-N^1-F-N^1]m-$ (I)

worin m = 2 bis 500,
B bedeutet -A-E-K-S-K-E-A- und zusätzlich gegebenenfalls -A-E-A'- bzw. -A'-E-A- ist,
worin S, K, -A-E-, - E-A-, -A-E-A'- bzw. -A'-E-A- und $-N^1-F-N^1-$ wie oben definiert sind, und der Anteil der Gruppe -A-E-A'- bzw. -A'-E-A- in der Gruppe B so gewählt sein kann, dass die Masse von -A-E-A'- bzw. -A'-E-A- von 0 bis 90 %, bevorzugt 0% oder 0,1 bis 50% der Masse des Polysiloxananteils S im Polymer beträgt.

Die erste Ausführungsform der Polyammonium-Polysiloxan Verbindungen betrifft bevorzugt lineare alkylenoxidmodifizierte polyquaternäre Polysiloxane der allgemeinen Formel (I'),

$$-[B-N^1-F-N^1],- \qquad (I')$$

worin

- m 2 bis 500,
- B -A-E-K-S-K-E-A-,
- S $-Si(R^1)_2-O[Si(R^1)_2-O]n-Si(R^1)_2-$
- $R^1$ $C_1$-$C_{22}$-Alkyl, $C_1$-$C_{22}$-Fluoralkyl oder Aryl,
- n 0 bis 1000,
- K ein zweiwertiger geradkettiger, cyclischer oder verzweigter $C_2$-$C_{20}$-Kohlenwasserstoffrest, der durch -O-, -NH-, $-NR^1-$, -C(O)-, -C(S) unterbrochen und mit -OH substituiert sein kann,
- E eine Polyalkylenoxideinheit der Struktur

$$-[CH_2CH_2O]_q-[CH_2CH(CH_3)O]_r - \text{mit},$$

q 1 bis 200,
r 0 bis 200 und
A $-CH_2C(O)O-$, $-CH_2CH_2C(O)O-$ oder - $CH_2CH_2CH_2C(O)O-$,
$N^1$ eine quarternäre Ammoniumstruktur $-(R^4)N^+(R^5)-$
$R^4$ ein einwertiger oder zweiwertiger geradkettiger, cyclischer oder verzweigter $C_1$-$C_{20}$-Kohlenwasserstoffrest darstellt, der durch O-, -NH, -C(O)-, -C(S)- unterbrochen und mit -OH substituiert sein kann,
$R^5$ $R^4$ oder eine Einfachbindung zu $R^4$ oder F darstellt,
F ein zweiwertiger oder vierwertiger geradkettiger, cyclischer oder verzweigter $C_2$-$C_{30}$- Kohlenwasserstoffrest darstellt, der durch -O-, -NH-, -N-, -C(O)-, -C(S)- , eine Siloxankette S, wobei für S die oben genannten Bezüge gelten, unterbrochen und mit -OH substituiert sein kann.

Die Möglichkeit einer vierwertigen Substruktur für F bedeutet, dass F ein verzweigtes oder Ringsystem mit den begrenzenden $N^1$ bilden kann, so dass F dann mit jeweils zwei Bindungen an der Quartärnierung von beiden begrenzenden $N^1$ beteiligt ist.

Zur näheren Illustration sei auf die Offenlegungsschrift WO 02/10257, insbesondere dort das Beispiel 1, verwiesen.

In einer weiteren Ausführungsform der Polyammonium-Polysiloxan Verbindungen bedeutet die Möglichkeit einer zweiwertigen Substruktur für $R^4$, dass es sich in diesen Fällen um eine cyclische Systeme bildende Struktur handelt, worin $R^5$ in diesem Fall eine Einfachbindung zu $R^4$ ist. Beispiele sind Morpholinyl- und Piperidinylstrukturen.
Bevorzugtere Ausführungsformen dieser so genannten ersten Ausführungsform der Erfindung sowie Verfahren zur Herstellung der genannten Polysiloxanverbindungen der Formel (I) bzw. (I') werden nachfolgend beschrieben.

$R^4$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$, - $CH_2CH_2NHCO-R^{14}$ oder $-CH_2CH_2CH_2NHCO-R^{14}$, worin
worin $R^{14}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann, ist.

$R^4$ und $R^5$ können wie vorstehend erwähnt auch gemeinsam eine cyclische Struktur der Formeln

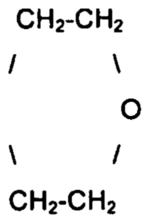

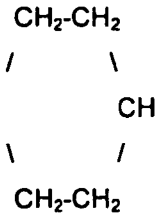

bilden.

Zu den bevorzugten Bedeutungen von $R^1$ in der so genannten ersten Ausführungsform der Polysiloxanverbindungen kann zu den vorstehenden Ausführungen verwiesen werden.

In der so genannten ersten Ausführungsform der Polysiloxanverbindungen ist $R^4$ bevorzugt ein einwertiger oder zweiwertiger geradkettiger, cyclischer oder verzweigter $C_1$-$C_{16}$-, bevorzugter $C_3$-$C_{16}$- Kohlenwasserstoffrest, der durch -O-, -NH-, -C(O)-, -C(S)- unterbrochen und mit -OH substituiert sein kann, bevorzugter ein $C_3$- $C_{16}$-Kohlenwasserstoffrest, der durch -O-, -NH-, -$NR^1$-, -C(O)-, -C(S)- unterbrochen und mit -OH substituiert sein kann, worin $R^1$ die obengenannte Bedeutung besitzt.

In der so genannten ersten Ausführungsform der Polysiloxanverbindungen ist F bevorzugt ein zweiwertiger oder vierwertiger geradkettiger, cyclischer oder verzweigter $C_2$-$C_{20}$-Kohlenwasserstoffrest, der durch -O-, -NH-, -N-, -C(O)-, -C(S), eine Siloxankette S, wobei für S die oben genannten Bezüge gelten, unterbrochen und mit -OH substituiert sein kann.

In der so genannten ersten Ausführungsform der Polysiloxanverbindungen ist K bevorzugt $-CH_2CH_2CH_2-$, $-(CH_2)_4-$, $-(CH_2)_6-$, $-CH_2CH_2CH_2OCH_2CH(OH)CH_2-$, und $-CH{=}CHCH_2-$.

In der so genannten ersten Ausführungsform der Polysiloxanverbindungen stellt $R^{14}$ bevorzugt unsubstituierte $C_5$-$C_{17}$-Kohlenwasserstoffreste dar, die sich von den entsprechenden Fettsäuren ableiten oder aber hydroxylierte $C_3$-$C_{17}$-Reste, die auf hydroxylierte Carbonsäuren, bevorzugt Saccharidcarbonsäuren zurückgeführt werden können.

In der so genannten ersten Ausführungsform sind die erfindungsgemäßen Polyammonium-Polysiloxanverbindungen bevorzugt, bei denen $R^{14}$ weiterhin stehen kann für hydroxylierte Reste aus der Gruppe bestehend aus

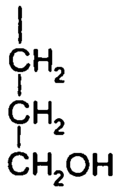

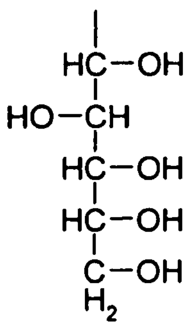

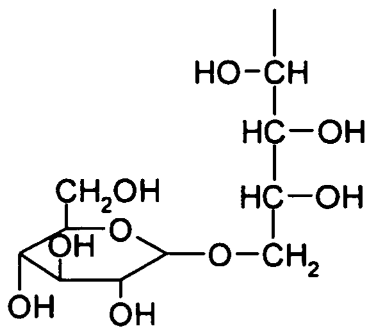

dar.

In der so genannten ersten Ausführungsform der Polysiloxanverbindungen ist m 2 bis 100, bevorzugt 2 bis 50.

In der so genannten ersten Ausführungsform der Polysiloxanverbindungen ist n 0 bis 1000, bevorzugt 0 bis 100, bevorzugter 0 bis 80 und besonders bevorzugt 10 bis 80.

In der so genannten ersten Ausführungsform der Erfindung ist q 1 bis 200, bevorzugt 1 bis 50, bevorzugter 2 bis 20 und besonders bevorzugt 2 bis 10.

In der so genannten ersten Ausführungsform der Erfindung ist r 0 bis 200, bevorzugt 0 bis 100, bevorzugter 0 bis 50 und noch bevorzugter 0 bis 20.

Zur Herstellung der erfindungsgemäßen Polysiloxan-Polyammonium Verbindungen sowohl dieser ersten Ausführungsform als auch aller weiteren bevorzugten Ausführungsformen der erfindungsgemäßen Polysiloxan- Polyammonium Verbindungen sei an dieser Stelle ganz explizit auf die Ausführungsbeispiele der Offenlegungsschrift WO 02/10257 verwiesen.

Eine besondere Ausführungsform der Erfindung (die im Folgenden als so genannte zweite Ausführungsform der Polysiloxanverbindungen bezeichnet wird) wird durch die Polysiloxan Verbindungen der allgemeinen Formel (II) dargestellt,

$R^2$-E-A-$N^2$ -K-S-K-$N^2$-A-E-$R^2$ (II)

worin
S, K, -A-E-, -E-A- und $R^2$ die oben genannten Bedeutungen aufweisen, und
$N^2$ ein organischer Rest, der mindestens eine quartäre Ammoniumgruppe enthält, der allgemeinen Formel -($R^8$)$N^+$($R^9$)- ist, worin

$R^8$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann,
$R^9$ ein einwertiger geradkettiger, cyclischer oder verzweigter $C_1$-$C_{20}$-Kohlenwasserstoffrest, der durch -O-, -NH-, -C (O)-, -C(S)- unterbrochen und mit -OH substituiert sein kann, oder eine Einfachbindung zu einem zweiwertigen Rest $R^8$ oder zu einem dreiwertigen Rest K darstellt, und die Reste $R^8$ und $R^9$ innerhalb der Polysiloxanverbindung der allgemeinen Formel (II) gleich oder verschieden voneinander sein können.

Bevorzugt handelt es sich bei den Polysiloxanverbindungen der zweiten Ausführungsform um ($\alpha$,$\omega$-Alkylenoxid- und polyquarternär modifizierte Polysiloxane der allgemeinen Formel (II'),

$R^{16}$- E-A-$N^2$-K-S-K-$N^2$-A-E-$R^{16}$ (II')

Worin die Bezeichnungen stehen für,

| | |
|---|---|
| S | -Si($R^1$)$_2$-O[-Si($R^1$)$_2$-O]$_n$Si($R^1$)- |
| mit $R^1$ | $C_1$-$C_{22}$-Alkyl, $C_1$-$C_{22}$-Fluoralkyl oder Aryl, |
| n bedeutet | 0 bis 1000, |
| K | ein zweiwertiger oder dreiwertiger geradkettiger, cyclischer oder verzweigter $C_2$-$C_{20}$-Kohlenwasserstoffrest, der durch -O-, -N-, -NH-, -$NR^1$-, -C(O)-, -C(S)-unterbrochen und mit -OH substituiert sein kann, |
| $N^2$ | eine quartäre Ammoniumstruktur -($R^8$)$N^+$($R^9$)- |
| | $R^8$ ein einwertiger oder zweiwertiger geradkettiger, cyclischer oder verzweigter $C_1$-$C_{20}$-Kohlenwasserstoffrest, der durch -O-, -NH-, C(O)-, -C(S)- unterbrochen und mit -OH substituiert sein kann, |
| | $R^9$ $R^8$ oder eine Einfachbindung zu K oder $R^8$, |
| A | $-CH_2C(O)O-$, $-CH_2CH_2C(O)O-$ oder $-CH_2CH_2CH_2C(O)O-$ |
| E | eine Polyalkylenoxideinheit der Struktur $-[CH_2CH_2O]_q-[CH_2CH(CH_3)O]_r-$ |
| | q 1 bis 200 |
| | r 0 bis 200 und |
| $R^{16}$ | H, geradkettiger, cyclischer oder verzweigter $C_1$-$C_{20}$-Kohlenwasserstoffrest, der durch -O-, oder -C(O)- unterbrochen und -OH substituiert und acetylenisch, olefinisch oder aromatisch sein kann. |

Die Möglichkeit einer dreiwertigen Substruktur für K bedeutet hier, dass K verzweigt sein kann und dann mit zwei Bindungen an der Quartärnierung von $N^2$ beteiligt ist. Die Möglichkeit einer zweiwertigen Substruktur für $R^8$ bedeutet, dass es sich in diesen Fällen um eine cyclische Systeme bildende Struktur handelt, wobei $R^9$ dann eine Einfachbindung zu $R^2$ ist.

$R^8$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$ $-CH_2CH_2NHCO-R^{17}$ oder $-CH_2CH_2CH_2NHCO-R^{17}$.
worin $R^{17}$ einen geradkettigen, cyclischen oder verzweigten $C_1$-$C_{18}$-Kohlenwasser-stoffrest, der durch -O-, -NH-, -C (O)-, -C(S)- unterbrochen und mit -OH substituiert sein kann, ist.
$R^8$ und $R^9$ können wie vorstehend erwähnt auch gemeinsam eine cyclische Struktur der Formeln

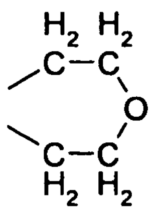

oder

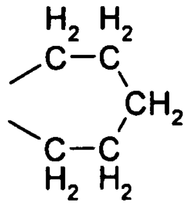

bilden.

Zu den bevorzugten Bedeutungen von $R^1$ in der so genannten zweiten Ausführungsform der Polysiloxanverbindungen kann zu den vorstehenden Ausführungen. verwiesen werden.

In der so genannten zweiten Ausführungsform der Polysiloxanverbindungen ist K bevorzugt ein zweiwertiger oder dreiwertiger geradkettiger, cyclischer oder verzweigter $C_3$-$C_{16}$-Kohlenwasserstoffrest, der durch -O-, -NH-, -$NR_1$-, -N-, -C(O)-, -C(S)- unterbrochen und mit -OH substituiert sein kann, worin $R^1$ wie vorstehend definiert ist.

Bevorzugt für K sind zum Beispiel Reste der folgenden Strukturen:

$-CH_2CH_2CH_2-$ $-CH_2CH_2CH_2OCH_2CHOHCH_2-$ oder

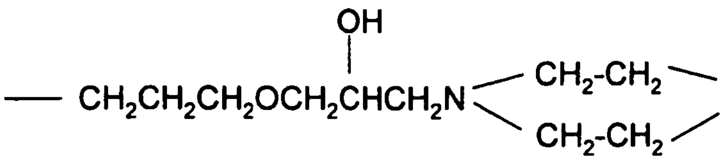

$R^8$ ist bevorzugt ein einwertiger oder zweiwertiger geradkettiger, cyclischer oder verzweigter $C_1$-$C_{16}$-Kohlenwasserstoffrest, der durch -O-, -NH-, -C(O), -C(S)- unterbrochen und mit -OH substituiert sein kann.

$R^{16}$ ist bevorzugt ein geradkettiger, cyclischer oder verzweigter $C_1$- $C_{18}$-Kohlen-wasserstoffrest, der durch -O- oder -C(O)- unterbrochen und mit - OH substituiert und acetylenisch oder olefinisch sein kann.

Weiterhin ist $R^{16}$ bevorzugt $C_5$-$C_{17}$-Alkyl, $-CH_2CH=CH_2$, $-CH_2CH(OH)CH_2OCH_2CH=CH_2$, $-CH_2CCH$, -C(O) $CH_3$, $-C(O)CH_2CH_3$.

$R^{17}$ stellt bevorzugt unsubstituierte $C_5$-$C_{17}$-Kohlenwasserstoffreste, die sich von den entsprechenden Fettsäuren ableiten oder aber hydroxylierte $C_3$-$C_{17}$-Reste, die auf hydroxylierte Carbonsäuren, bevorzugt auf Saccharidcarbonsäuren zurückgeführt werden können, dar.

$R^{17}$ wird besonders bevorzugt aus der Gruppe aus

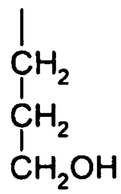

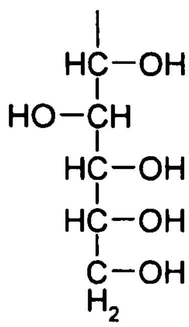

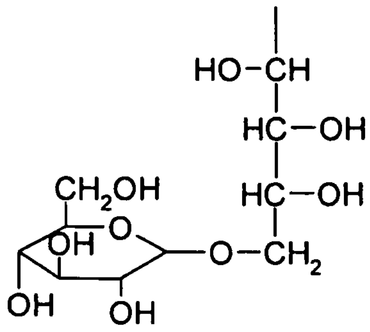

ausgewählt.

In der so genannten zweiten Ausführungsform der Polysiloxanverbindungen ist n bevorzugt 0 bis 200, bevorzugter 0 bis 80, besonders bevorzugt 10 bis 80.

In der so genannten zweiten Ausführungsform der Polysiloxanverbindungen ist q bevorzugt 1 bis 50, bevorzugter 2 bis 20 und besonders bevorzugt 2 bis 10.

In der so genannten zweiten Ausführungsform der Polysiloxanverbindungen ist r bevorzugt 0 bis 100 und bevorzugter 0 bis 50.

In der so genannten zweiten Ausführungsform der Erfindung ist r bevorzugt 0 bis 20 und bevorzugter 0 bis 10.

Zur Herstellung der erfindungsgemäßen Polysiloxan-Verbindungen der so genannten zweiten Ausführungsform sei auf die Ausführungen zur ersten bevorzugten Ausführungsform verwiesen.

Eine besondere Ausführungsform der Polyammonium-Polysiloxan Verbindungen (die im Folgenden als so genannte dritte Ausführungsform der Polysiloxane bezeichnet wird) wird durch die Polysiloxan Verbindungen der allgemeinen Formel (III) dargestellt:

$$-[K-S-K-N^3]_m- \qquad (III)$$

in der S, K und m wie oben definiert sind,

$N^3$ ein organischer Rest, der mindestens eine quartäre Ammoniumgruppe enthält, der allgemeinen Formel

$-(R^{10})-N+(R^{11})-$

ist, worin $R^{10}$ ein einwertiger geradkettiger, cyclischer oder verzweigter $C_1$- $C_{30}$-Kohlenwasserstoffrest, der durch -O-, -NH-, -C(O)-, -C(S)- unterbrochen und mit -OH substituiert sein kann oder eine Einfachbindung zu K darstellt,
$R^{11}$ steht für $-A-E-R^2$, worin $-A-E-R^2$ die oben genannte Bedeutung aufweist.

Bevorzugt handelt es sich bei den Polysiloxanverbindungen der dritten Ausführungsform um Alkylenoxidmodifizierte polyparternäre Polysiloxane der allgemeinen Formel (III'),

$$-[K\text{-}S\text{-}K\text{-}N^3]m- \qquad (III'),$$

in der m 2 bis 500 ist,
S bedeutet $-Si(R^1)_2-O[-Si(R^1)_2-O]_n-Si(R^1)_2-$
mit $R^1$ $C_1$-$C_{22}$-Alkyl, $C_1$-$C_{22}$-Fluoralkyl oder Aryl,
n = 0 bis 1000,
$N^3$ eine quartäre Ammoniumstruktur

$$-(R^{10})N^+(R^{11})-$$

worin $R^{10}$ ein einwertiger oder zweiwertiger geradkettiger, cyclischer oder verzweigter $C_1$-$C_{30}$-Kohlenwasserstoffrest, der durch -O-, -NH-, -C(O)-, -C(S)-unterbrochen und mit -OH substituiert sein kann oder eine Einfachbindung zu K darstellt,
$R^3$ -A-E - ist, mit
A für $-CH_2C(O)O-$, $-CH_2CH_2C(O)O-$ oder - $CH_2CH_2CH_2C(O)O-$ und
E für eine Polyalkylenoxideinheit der Struktur

$$-[CH_2CH_2O]_q-[CH_2CH(CH_3)O]_r-R^{18}$$

q von 1 bis 200,
r von 0 bis 200,
$R^{18}$ für H, geradkettiger, cyclischer oder verzweigter $C_1$-$C_{20}$-Kohlenwasserstoffrest, der durch -O-, oder -C(O)- unterbrochen -und mit -OH substituiert und acetylenisch, olefinisch oder aromatisch sein kann, sowie
K ist ein zweiwertiger oder dreiwertiger geradkettiger, cyclischer oder verzweigter $C_2$-$C_{40}$-Kohlenwasserstoffrest, der durch -O-, -NH-, - $NR^1$-, -N-, -C(O)-, -C(S)- unterbrochen und mit -OH substituiert sein kann oder eine quartäre Ammonium-struktur $N^5$ enthält, mit
$N^5$ in der Bedeutung von $-(R^{19})N^+(R^{20})-$
$R^{19}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann oder eine Einfachbindung zu $R^{10}$ darstellt, und $R^{20}$ -A-E- ist, das wie oben definiert ist.

Zur Herstellung der bevorzugten Ausführungsformen der so genannten dritten Ausführungsform der Polysiloxanverbindungen sei wie bereits zuvor explizit auf die Ausführungsbeispiele der Offenlegungsschrift WO 02/10257 verwiesen.

$R^{10}$ und $R^{19}$ sind unabhängig voneinander bevorzugt $-CH_3$, $-CH_2CH_3$, $-(CH_2)_2CH_3$, $-(CH_2)_3CH_3$, $-(CH_2)_5CH_3$, $-CH_2CH_2OH$, $-CH_2CH_2NHCOR^{21}$ oder $-CH_2CH_2CH_2NHCOR^{21}$, worin $R^{21}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann, ist.

In einer Ausführungsform der so genannten dritten Ausführungsform der Polysiloxanverbindungen handelt es sich bei einer zweiwertigen Substruktur für $R^{10}$ um eine ein cyclisches System bildende Struktur, wobei $R^{10}$ dann eine Einfachbindung zu K besitzt, bevorzugt zu einer tertiären Aminostruktur oder aber zur quartären Struktur $N^5$ über $R^{19}$.

Zu den bevorzugten Bedeutungen von $R^1$ in der so genannten dritten Ausführungsform der Polysiloxane kann zu den obigen Ausführungen verwiesen werden.

Bevorzugt ist $R^{10}$ ein einwertiger oder zweiwertiger geradkettiger, cyclischer oder verzweigter $C_1$-$C_{25}$-Kohlenwasserstoffrest, der durch -O-, -NH-, -C(O)-, -C(S)- unterbrochen und mit -OH substituiert sein kann.

Bevorzugt ist $R^{19}$ ein einwertiger oder zweiwertiger geradkettiger, cyclischer oder verzweigter $C_1$-$C_{25}$-Kohlenwasserstoffrest, der durch -O-, -NH-, -C(O)-, -C(S)- unterbrochen und mit -OH substituiert sein kann.

In der so genannten dritten Ausführungsform der Polysiloxanverbindungen ist K weiterhin bevorzugt ein zweiwertiger oder dreiwertiger geradkettiger, cyclischer oder verzweigter $C_3$-$C_{30}$-Kohlenwasserstoffrest, der durch -O-, -NH-, $-NR^1-$, -N-, -C(O)-, -C(S)- unterbrochen und mit -OH substituiert sein kann, noch bevorzugter ist K

$-CH_2CH_2CH_2OCH_2CHOHCH_2-$,

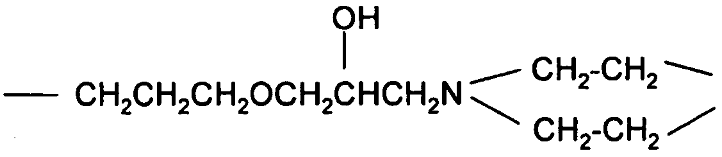

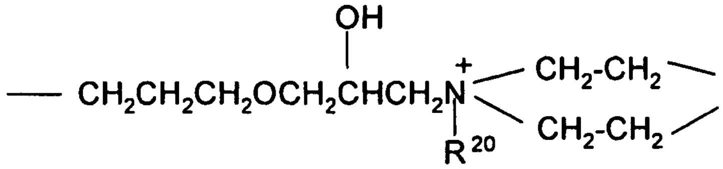

worin $R^{20}$ wie oben definiert ist.

In der so genannten dritten Ausführungsform der Polysiloxane ist $R^2$ bzw. $R^{18}$ bevorzugt ein geradkettiger, cyclischer oder verzweigter $C_1$-$C_{18}$Kohlenwasser-stoffrest, der durch -O- oder -C(O)- unterbrochen und -OH substituiert und acetylenisch oder olefinisch sein kann. Bevorzugter ist $R^2$ bzw. $R^{18}$ $C_1$-$C_6$-Alkyl, $-CH_2CH{=}CH_2$, $-CH_2CH(OH)CH_2OCH_2CH{=}CH_2$, $-CH_2CCH$, $-C(O)CH_3$ oder $-C(O)CH_2CH_3$.

Bevorzugt ist $R^{21}$ ein unsubstituierter $C_5$-$C_{17}$-Kohlenwasserstoffrest, der sich von den entsprechenden Fettsäuren ableitet oder aber hydroxylierte $C_3$-$C_{17}$-Reste aufweist, und aus der Gruppe von hydroxylierten Carbonsäuren, bevorzugt Saccharidcarbonsäuren stammt.

So ist $R^{21}$ beispielsweise:

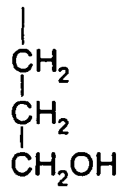

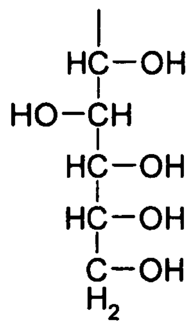

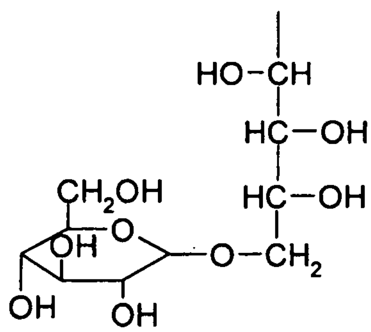

In der so genannten dritten Ausführungsform der Polysiloxane ist m bevorzugt 2 bis 100, und besonders bevorzugt 2 bis 50, n ist 0 bis 100, bevorzugt 0 bis 80, und besonders bevorzugt 10 bis 80, q ist 1 bis 50, bevorzugt 2 bis 50 besonders bevorzugt 2 bis 20, und noch bevorzugter ist q 2 bis 10, r ist 0 bis 100, bevorzugt 0 bis 50, besonders bevorzugt 0 bis 20, und noch bevorzugter ist r 0 bis 10.

Zur Herstellung der erfindungsgemäßen in der Wirkstoffkombination zu verwendenden Polysiloxan-Verbindungen der so genannten dritten Ausführungsform wird zweckmäßig wiederum auf die Ausführungsbeispiele der Offenlegungsschrift WO 02/10257 verwiesen.

Eine besondere Ausführungsform der Polysiloxane (die im Folgenden als so genannte vierte Ausführungsform der erfindungsgemäß zu verwendenden Polysiloxane bezeichnet wird) wird durch die Polysiloxanverbindungen der allgemeinen Formel (IV) dargestellt:

$$-[N^4\text{-}K\text{-}S\text{-}K\text{-}N^4\text{-}A\text{-}E\text{-}A']_m \text{ bzw. } -[N^4\text{-}K\text{-}S\text{-}K\text{-}N^4\text{-}A'\text{-}E\text{-}A]_m- \qquad (IV)$$

worin m, K, S, -A-E-A'- und -A'-E-A- wie oben definiert sind, und
$N^4$ ein organischer Rest, der mindestens eine quartäre Ammoniumgruppe enthält, der allgemeinen Formel $-(R^{12})N^+(R^{13})-$ ist, worin $R^{12}$ ein einwertiger oder zweiwertiger geradkettiger, cyclischer oder verzweigter $C_1$-$C_{20}$-Kohlenwasserstoffrest ist, der durch -O-, -NH-, -C(O)-, -C(S)- unterbrochen und mit -OH substituiert sein kann,

$R^{13}$ die Bedeutungen von $R^{12}$ aufweisen kann, oder eine Einfachbindung zu K oder $R^{12}$ darstellt, und die Reste $R^{12}$ und $R^{13}$ gleich oder verschieden voneinander sein können.

Bevorzugt handelt es sich bei den Polysiloxanverbindungen der vierten Ausführungsform um Alkylenoxidmodifizierte polyquarternäre Polysiloxane der allgemeinen Formel (IV'),

$$-[N^4\text{-K-S-K-}N^4\text{-A-E-A}]m- \quad (IV')$$

worin m = 2 bis 500,

S $-Si(R^1)_2-O[-Si(R^1)_2-O]-Si(R^1)_2-$, worin
$R^1$ steht für $C_1$-$C_{22}$-Alkyl, $C_1$-$C_{22}$-Fluoralkyl oder Aryl,
n 0 bis 1000,

K einen zweiwertigen. oder dreiwertigen geradkettigen, cyclischen oder verzweigten $C_2$-$C_{20}$-Kohlenwasserstoffrest darstellt, der durch -O-, -NH-, $-NR^1$-, -N-, -C(O)-, -C(S)- unterbrochen und mit -OH substituiert sein kann,

$N^4$ eine quartäre Ammoniumstruktur $-(R^{12})N^+(R^{13})$- ist, worin $R^{12}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann, $R^{13}$ steht für $R^{12}$ oder eine Einfachbindung zu K oder $R^{12}$,

A ist $-CH_2C(O)O-$, $-CH_2CH_2C(O)O-$ oder $-CH_2CH_2CH_2C(O)O-$

E ist eine Polyalkylenoxideinheit der Struktur $-[CH_2CH_2O]_q-[CH_2CH(CH_3)O]_r-$
mit q = 1 bis 200 und
r = 0 bis 200.

Zu den Herstellungsverfahren sei auf das bisher ausgeführte verwiesen.

Bevorzugtere Ausführungsformen dieser so genannten vierten Ausführungsform Polysiloxane der Formel (IV) bzw. (IV') werden nachfolgend beschrieben.

Die Möglichkeit einer dreiwertigen Substruktur für K bedeutet, dass K verzweigt sein kann und dann mit zwei Bindungen an der Quartärnierung von $N^4$ beteiligt sein kann.

Die Möglichkeit einer zweiwertigen Substruktur für $R^{12}$ bedeutet, dass es sich in diesen Fällen um eine cyclische Systeme bildende Struktur handelt, wobei $R^{13}$ dann eine Einfachbindung zu $R^{12}$ ist.

$R^{12}$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$, $-CH_2CH_2NHCOR^{22}$ oder-$CH_2CH_2CH_2NHCOR^{22}$,
worin $R^{22}$ einen geradkettigen, cyclischen oder verzweigten $C_1$-$C_{18}$Kohlenwasser-stoffrest, der durch -O-, -NH-, -C(O)-, -C(S)- unterbrochen und mit -OH substituiert sein kann, ist.

$R^{12}$ und $R^{13}$ können wie vorstehend erwähnt auch gemeinsam eine cyclische Struktur der Formeln

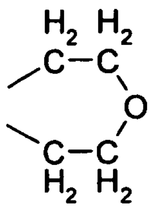
oder
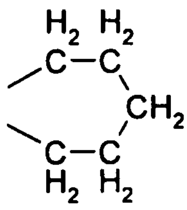

bilden.

Zu den bevorzugten Bedeutungen von $R^1$ in der so genannten vierten Ausführungsform der Polysiloxane kann auf die vorstehenden Ausführungen verwiesen werden.

Bevorzugt ist $R^{12}$ ein einwertiger oder zweiwertiger geradkettiger, cyclischer oder verzweigter $C_1$-$C_{16}$-Kohlenwasserstoffrest, der durch -O-, -NH-, -C(O)-, -C(S)- unterbrochen und mit -OH substituiert sein kann.

In der so genannten vierten Ausführungsform, ist K bevorzugt ein zweiwertiger oder dreiwertiger geradkettiger, cyclischer oder verzweigter $C_3$-$C_{16}$- Kohlenwasserstoffrest, der durch -O-, -NH-, $-NR^1$-, -N-, -C(O)-, -C(S)- unterbrochen und mit -OH substituiert sein kann, besonders bevorzugt ist K $-CH_2CH_2CH_2-$, $-CH_2CH_2CH_2OCH_2CHOHCH_2-$ oder

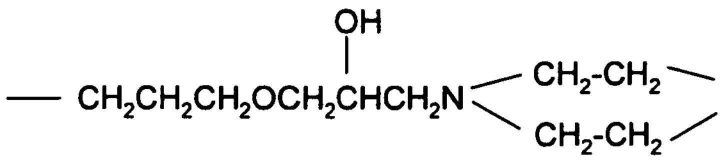

Bevorzugt ist $R^{22}$ ein unsubstituierter $C^5$-$C^{17}$-Kohlenwasserstoffrest, der sich von den entsprechenden Fettsäuren ableitet oder aber hydroxylierte $C^3$-$C^{17}$-Reste aufweist, die auf hydroxylierte Carbonsäuren, bevorzugt Saccharidcarbonsäuren zurückgeführt worden können.

Bevorzugter ist $R^{22}$:

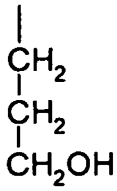

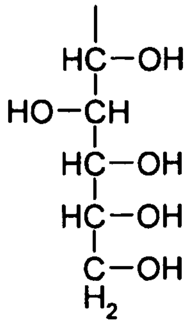

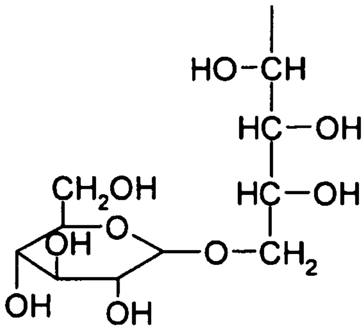

m ist bevorzugt 2 bis 100, und besonders bevorzugt 2 bis 50. n ist 0 bis 100, bevorzugt 0 bis 80, und besonders bevorzugt 10 bis 80. q ist 1 bis 50, bevorzugt 2 bis 50, und besonders bevorzugt 2 bis 20, noch bevorzugter ist q 2 bis 10. r ist 0 bis 100, bevorzugt 0 bis 50, und besonders bevorzugt 0 bis 20, noch bevorzugter ist r 0 bis 10.

Der Begriff "$C_1$-$C_{22}$-Alkyl oder $C_1$-$C_{30}$-Kohlenwasserstoffrest", wie er vorstehend verwendet wird, bedeutet im Rahmen der vorliegenden Erfindung aliphatische Kohlenstoffwasserstoffverbindungen mit 1 bis 22 Kohlenstoffatomen bzw. 1 bis 30 Kohlenstoffatomen die geradkettig oder verzweigt sein können. Beispielhaft seien Methyl, Ethyl, Propyl, n-Butyl, Pentyl, Hexyl, Heptyl, Nonyl, Decyl, Undecyl, iso Propyl, Neopentyl, und 1,2,3 Trimethylhexyl aufgeführt.

Der Begriff "$C_1$-$C_{22}$-Fluoralkyl" bedeutet, wie er vorstehend verwendet wird, im Rahmen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2 Triflourbutyl aufgeführt.

Der Begriff "Aryl ", wie er vorstehend verwendet wird, bedeutet im Rahmen der vorliegenden Erfindung unsubstituierte oder ein oder mehrfach mit OH, F, Cl, CF3 $C_1$-$C_6$-Alkyl, $C_1$-$C_6$-Alkoxy, $C_3$-$C_7$- Cycloalkyl, $C_2$-$C_6$-Alkenyl oder Phenyl substituiertes Phenyl. Der Ausdruck kann gegebenenfalls auch Naphthyl bedeuten.

Im Rahmen dieser vierten Ausführungsform hat es sich als besonders bevorzugt erwiesen, wenn $N^4$ steht für $-(R^{12})N^+(R^{13})-$ wobei $R^{12}$ steht für eine $C_1$- bis $C_4$-Alkylgruppe und $R_{13}$ steht für eine Einfachbindung zu K.

Weiterhin hat es sich als besonders bevorzugt erwiesen, wenn K steht für einen Rest

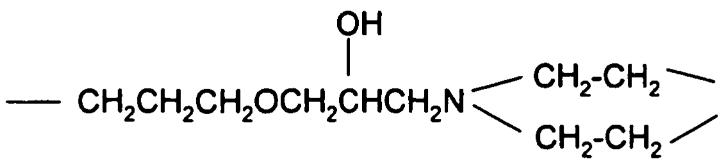

Ebenso kann es erfindungsgemäß bevorzugt sein, wenn S steht für eine Gruppe $-Si(R^1)_2-O[-Si(R^1)_2-O]_n-Si(R^1)_2-$,
worin $R^1$ bedeutet $C_1$-$C_{22}$-Alkyl, $C_1$-$C_{22}$-Fluoralkyl oder Aryl, vorzugsweise Phenyl, n = 0 bis 1000, und wenn mehrere Gruppen S in der Polysiloxanverbindung vorliegen, diese gleich oder verschieden sein können. Weiterhin hat es sich erfindungsgemäß als bevorzugt erwiesen, wenn $R^1$ jeweils für eine Methylgruppe steht.

Ausserdem kann es erfindungsgemäß bevorzugt sein, wenn A steht für eine Gruppe $-CH_2CH_2C(O)O-$ , A' steht für eine Gruppe $-CH_2CH_2C(O)-$, und E steht für eine Polyalkylengruppe der allgemeinen Formel $-[CH_2CH_2O]_q-$ mit q =

1 bis 200 wobei das endständige Sauerstoffatom der Gruppe A an die endständige $-CH_2$-Gruppe der Gruppe E, und das endständige Carbonylkohlenstoffatom der Gruppe A' an das endständige Sauerstoffatom Gruppe E jeweils unter Ausbildung von Estergruppen binden.

Eine besondere Ausführungsform der erfindungsgemäßen Polysiloxane (die im Folgenden als so genannte fünfte Ausführungsform der Polysiloxane bezeichnet wird) wird durch die Polysiloxane der allgemeinen Formel (V) dargestellt:

$$[-N^5-F^1-N^5-Y-]_m$$

worin

Y eine Gruppe der Formel -K-S-K- und -A-E-A'- bzw. -A'-E-A- ist,

worin m, K, S, -A-E-A'- und -A'-E-A- wie oben definiert sind, die Gruppen K, S, -A-E-A'- und -A'-E-A- innerhalb der Polysiloxane der allgemeinen Formel (V) gleich oder verschieden voneinander sein können, und das molare Verhältnis der Gruppe -K-S-K- und der Gruppe -A-E-A'- bzw. -A'-E-A- in der Polysiloxanverbindung der allgemeinen Formel (V) von 100: 1 bis 1: 100 ist,

$N^5$ eine Ammoniumgruppe der allgemeinen Formel $-(R^{23})N^+(R^{24})-$ ist,worin
$R^{23}$ Wasserstoff, ein einwertiger oder zweiwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann,
$R^{24}$ Wasserstoff, ein einwertiger geradkettiger, cyclischer oder verzweigter $C_1$-$C_{20}$-Kohlenwasserstoffrest darstellt, der durch -O-, -NH-, - C(O)-, C(S)- unterbrochen und mit -OH substituiert sein kann, oder eine Einfachbindung zu einem zweiwertigen Rest $R^{23}$ darstellt, und die Reste $R^{23}$ und $R^{24}$ innerhalb der Gruppe $-N^5-F^1-N^5-$ sowie in der Polysiloxanverbindung gleich oder verschieden voneinander sein können,
$F^1$ ein zweiwertiger geradkettiger, cyclischer oder verzweigter Kohlenwasserstoffrest darstellt, der durch -O-, -NH-, -N-, -C(O)- oder -C(S)- oder durch eine Gruppe -E- unterbrochen sein kann, worin E wie oben definiert ist, und worin eine Mehrzahl von $N^5$ und $F^1$ jeweils gleich oder verschieden voneinander sein können.

Das molare Verhältnis der Gruppe -K-S-K- und der Gruppe -A-E-A'- bzw. -A'-E-A- in der Polysiloxanverbindung der allgemeinen Formel (V) liegt zwischen 100: 1 und 1: 100. Dieses molare Verhältnis kann wie in der Offenlegungsschrift WO 02/10257 gezeigt, durch die Wahl des molaren Verhältnisses der Ausgangsverbindungen, insbesondere des Verhältnisses der erfindungsgemäß bevorzugt verwendeten ($\alpha,\omega$-Halogencarbonsäurepolyalkylenoxidester-Verbindungen und der Polysiloxan-Bisepoxid-Verbindungen gesteuert werden. Die Eigenschaften der Produkte hängen wesentlich vom verwendeten Verhältnis der Ausgangsmaterialien, sowie der Länge der darin enthaltenen Polyalkylenoxid- bzw. Polysiloxanblöcke ab. In einer bevorzugten Ausführungsform der so genannten fünften Ausführungsform der Polysiloxane ist K ein zweiwertiger Kohlenwasserstoffrest mit mindestens 4 Kohlenstoffatomen, der eine Hydroxylgruppe aufweist und der durch ein Sauerstoffatom unterbrochen sein kann.

In einer bevorzugten Ausführungsform der so genannten fünften Ausführungsform der Polysiloxane ist F1 ein zweiwertiger geradkettiger, cyclischer oder verzweigter $C_2$-$C_{30}$-Kohlenwasserstoffrest, der durch -O-, -NH-, -N-, -C(O)-, -C(S)- oder durch eine Gruppe -E- unterbrochen sein kann, worin E wie oben definiert ist, und worin die Kohlenstoffatome, die aus dem Rest E resultieren, nicht zu den 2 bis 30 Kohlenstoffatomen des $C_2$-$C_{30}$ Kohlenwasserstoffrest gezählt werden.

In einer weiteren bevorzugten Ausführungsform der so genannten fünften Ausführungsform der Erfindung ist

$$-N^5 -F^1-N^5-$$

eine Gruppe der Formel:

$$-N(R^{25}R^{26})+-F^2-N(R^{25}R^{26})^+-$$

worin

$R^{25}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann, besonders bevorzugt Methyl ist,
$R^{26}$ ein einwertiger geradkettiger, cyclischer oder verzweigter $C_1$-$C_{20}$-Kohlenwasserstoffrest, der durch -O-, -NH-, -C(O)-, -C(S) unterbrochen und mit -OH substituiert sein kann, besonders bevorzugt Methyl ist, oder eine Einfachbindung zu einem zweiwertigen Rest $R^{25}$ darstellt, und die Reste $R^{25}$ und $R^{26}$ innerhalb der Gruppe $-N^5-F^2-N^5-$ sowie in der Polysiloxanverbindung gleich oder verschieden voneinander sein können, und
$F^2$ ein zweiwertiger geradkettiger, cyclischer oder verzweigter Kohlenwasserstoffrest ist, der durch -O-, -NH-, -N-,

-C(O)-, -C(S)- unterbrochen sein kann.

In einer noch bevorzugteren Ausführungsform ist $F^2$ eine verzweigte, bevorzugt geradkettige $C_1$-$C_6$-Alkandiyl-Gruppe, worunter eine 1,6-Hexandiyl- (bzw. Hexamethylen-) Gruppe bevorzugt ist.

In einer weiteren bevorzugten Ausführungsform. der so genannten fünften Ausführungsform der Polysiloxanverbindungen ist

$-N^5-F^1-N^5-$

eine Gruppe der Formel:

$-N(R^{27}R^{28})^+-F^3-N(R^{27}R^{28})^+-$

worin

$R^{27}$ und $R^{28}$ jeweils Wasserstoff, $C_1$-$C_6$-Alkyl oder Hydroxy($C_1$-$C_6$)alkyl, bevorzugt Wasserstoff, Methyl oder $-CH_2CH_2OH$ sind, und

$F^3$

ein zweiwertiger geradkettiger, cyclischer oder verzweigter Kohlenwasserstoffrest ist, der durch eine Gruppe -E- unterbrochen ist, worin E wie oben definiert ist.

$F^3$ ist besonders bevorzugt eine Gruppe der Formel

-D-E-D-

worin E wie oben definiert ist und D jeweils eine Einfachbindung oder eine geradkettige oder verzweigte $C^1$-$C^6$-Alkandiylgruppe ist, mit der Maßgabe, das D keine Einfachbindung ist, wenn es an ein endständiges Sauerstoffatom der Gruppe E bindet.

Bevorzugt wird die Gruppe -D-E-D- durch eine Gruppe der Formel

$-D-(OCH_2CH_2)_v(OCH_2CH(CH_3))_w-O-D-$

dargestellt, worin D eine geradkettige oder verzweigte $C_1$ -$C_6$-Alkandiylgruppe ist und r und q wie oben definiert sind. In der Gruppe $-D-(OCH_2CH_2)_q(OCH_2CH(CH_3))_r-O-D-$ können die Ethylenoxid- und Propylenoxideinheiten beliebig angeordnet sein, z.B. als statistische Copolymereinheit oder als Blockcopolymereinheit.

v ist bevorzugt 1 bis 100, bevorzugter 1 bis 70, noch bevorzugter 1 bis 40.

w ist bevorzugt 0 bis 100, bevorzugter 0 bis 70, noch bevorzugter 0 bis 40.

In einer weiteren bevorzugten Ausführungsform der so genannten fünften Ausführungsform der Erfindung wird die Gruppe

$-N^5-F^1-N^5$

durch eine Gruppe der Formel:

$-N^+R^{25}R^{26}-F^2-N^+R^{25}R^{26}-$

und eine Gruppe der Formel:

$-N^+R^{27}R^{28}-F^3-N^+R^{27}R^{28}-$

dargestellt, worin die Substituenten jeweils die vorstehenden Bedeutungen aufweisen.

Dies bedeutet, das die Polysiloxanverbindungen der allgemeinen Formel (V) aus zwei verschiedenen Typen der Gruppe $-N^5-F^1-N^5-$ aufgebaut sind.

In dieser Ausführungsform beträgt das molare Verhältnis der Gruppe

$-N^+R^{25}R^{26}-F^2-N^+R^{25}R^{26}-$

zur Gruppe

$-N^+R^{27}R^{28}-F^3-N^+R^{27}R^{28}-$

zweckmäßig 70 : 30 bis 95 : 5, bevorzugt 80 : 20 bis 90 : 10.

Die Polysiloxanverbindungen der allgemeinen Formel (V) können cylisch oder linear sein. Im Falle der linearen Verbindungen resultieren die endständigen Gruppen entweder aus den für die Herstellung verwendeten unten beschriebenen bifunktionellen Monomeren oder deren funktionalisierten Derivaten oder aus Monoaminen, die während der Polymerisation als Kettenabbruchmittel zugesetzt werden. Die aus der Verwendung der Monoamin-Kettenabbruchmittel resultierenden terminalen Gruppen liegen bevorzugt als Ammoniumgruppen, entweder durch Quartärnierung oder Protonierung vor.

In einer weiteren bevorzugten Ausführungsform der so genannten fünften Ausführungsform der Polysiloxane steht K für eine der Gruppen der Formel:

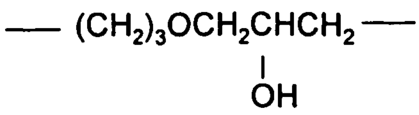

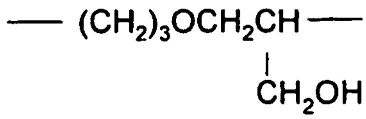

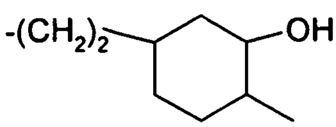

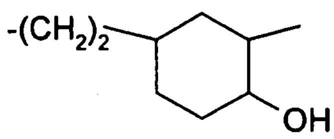

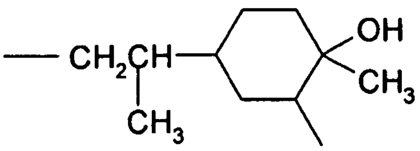

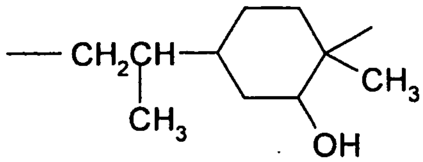

In der so genannten fünften Ausführungsform der Polysiloxane liegt q bevorzugt im Bereich von 1 bis 50, insbesondere 2 bis 50, speziell 2 bis 20 und ganz speziell 2 bis 10, und r liegt im Bereich von 0 bis 100, insbesondere 0 bis 50, speziell 0 bis 20 und ganz speziell 0 bis 10.

In der so genannten fünften Ausführungsform der Erfindung wird der organische oder anorganische Säurerest zur Neutralisation der aus der(n) Ammoniumgruppe(n) resultierenden Ladungen zweckmäßig ausgewählt aus anorganischen Resten, wie Chlorid, Bromid, Hydrogensulfat, Sulfat, bzw. organischen Resten, wie Acetat, Propionat, Octanoat, Decanoat, Dodecanoat, Tetradecanoat, Hexadecanoat, Octadecanoat und Oleat, wobei wie eingangs erwähnt Chlorid und Bromid bevorzugt aus der Umsetzung der Alkylhalogenidgruppen mit Amingruppen resultieren.

Weiterhin liegen die Polysiloxane der fünften Ausführungsform in protonierter Form als Aminsalze oder als Amine vor.

Die Polysiloxane der fünften Ausführungsform der Erfindung werden zweckmäßig hergestellt durch eines der Verfahren, welche in der Offenlegungsschrift WO 02/10257 beschrieben sind.

Die vorstehend beschriebenen Polyammonium-Polysiloxan Verbindungen können beispielsweise unter der Handelsbezeichnung Baysilone® von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911. Weiterhin bevorzugt sind die Polyammonium-Polysiloxan Verbindungen, wie sie in Beispiel 8 der WO 02/10257 beschrieben sind. Dabei sollen erfindungsgemäß auch solche Verbindungen besonders bevorzugt sein, die sich von der in Beispiel 8 der WO 02/10257 genannten Verbindung nur durch eine Variation der Länge der Silikonkette und/oder eine Variation der Länge der Polyetherkette unterscheiden.

Die vorstehend beschriebenen Polyammonium-Polysiloxan Verbindungen werden erfindungsgemäß vorzugsweise in einer Menge von 0,01 bis 15 Gew.% , vorzugsweise 0,05 bis 10, besonders bevorzugt 0,1 bis 5,0 Gew.%, ganz besonders bevorzugt von 0,1 bis 2,5 Gew.% jeweils in Bezug auf die Gesamtzusammensetzung verwendet.

Die amphoteren Tenside der Formel (I) sind die zweite Komponente der erfindungsgemäßen Kombination. In einer bevorzugten Ausführungsform steht der Rest R gemäß Formel (I) für eine lineare ($C_{12}$ bis $C_{18}$)-Alkylgruppe. Besonders bevorzugte Verbindungen der Formel (I) werden unter der INCI-Bezeichnung Disodium Cocoamphodipropionate mit den Handelsnamen Miranol® C2M SF conc. (Rhodia), Amphoterge® K-2 (Lonza) und Monateric® CEM-38 (Unichema) vermarktet. Dabei leitet sich die Gruppe R-CO- gemäß Formel (I) von Fettsäuren des Kokosnußöls ab.

Die Verbindungen der Formel (I) sind in dem erfindungsgemäßen Mittel bevorzugt in einer Menge von 0,05 bis 20 Gew.-%, besonders bevorzugt von 0,1 bis 15 Gew.-%, jeweils bezogen auf das Gewicht des Mittels, enthalten.

Die zweite Komponente der Wirkstoffkombination ist ein amphoteres und/oder ein kationisches Polymer.

Unter dem Begriff amphotere Polymere werden solche Polymere verstanden,

- die im Molekül sowohl freie Aminogruppen als auch freie -COOH- oder $SO_3H$-Gruppen enthalten und zur Ausbildung innerer Salze befähigt sind,
- zwitterionische Polymere, die im Molekül quartäre Ammoniumgruppen und $-COO^-$-Gruppen oder $-SO_3^-$-Gruppen enthalten, sowie
- Polymere, die -COOH-Gruppen oder $SO_3H$-Gruppen und quartäre Ammoniumgruppen enthalten.

Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß bevorzugte amphotere und/oder kationische Polymere sind solche Polymerisate, in denen sich eine kationische Gruppe ableitet von mindestens einem der folgenden Monomere:

(M1) Monomeren mit quartären Ammoniumgruppen der allgemeinen Formel (II),

$$R^1\text{-}CH{=}CR^2\text{-}CO\text{-}Z\text{-}(C_nH_{2n})\text{-}N^{(+)}R^3R^4R^5\ A^{(-)} \qquad (II)$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist,

(M2) Monomeren mit quartären Ammoniumgruppen der allgemeinen Formel (III),

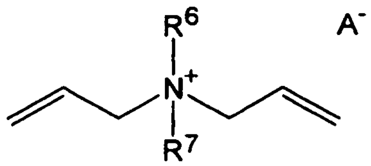

**(III)**

worin $R^6$ und $R^7$ unabhängig voneinander stehen für eine ($C_1$ bis $C_4$)-Alkylgruppe, insbesondere für eine Methylgruppe und $A^-$ das Anion einer organischen oder anorganischen Säure ist.

Wenn sich eine kationische Gruppe der amphoteren bzw. kationischen Polymerisate vom Monomer des Typs (M1) ableitet, stehen in Formel (II) die Reste $R^3$, $R^4$ und $R^5$ bevorzugt für Methylgruppen, Z ist bevorzugt eine NH-Gruppe und $A^{(-)}$ bedeutet bevorzugt ein Halogenid-, Methoxysulfat- oder Ethoxysulfat-Ion. Besonders bevorzugt ist es in diesem Falle Acrylamidopropyl-trimethyl-ammoniumchlorid als Monomer (M1) zu verwenden.

In Formel (III) steht $A^-$ bevorzugt für ein Halogenidion, insbesondere für Chlorid oder Bromid.

Bevorzugte erfindungsgemäße amphotere Polymere sind Polymere, deren anionische Gruppe sich von mindestens einem Monomeren der Formel (IV) ableitet

(M3) monomeren Carbonsäuren der allgemeinen Formel (IV) bzw. deren Salze mit einer organischen oder anorganischen Säure,

$R^8$-CH=C$R^8$-COOH(IV)

in denen $R^8$ und $R^9$ unabhängig voneinander Wasserstoff oder Methylgruppen sind.

Als Monomeres (M3) wird für die erfindungsgemäß bevorzugten amphoteren Polymerisate Acrylsäure verwendet.

Besonders bevorzugte amphotere Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Die amphoteren Polymere können sowohl direkt als auch in Salzform, die durch Neutralisation der Polymerisate, beispielsweise mit einem Alkalihydroxid, erhalten wird, erfindungsgemäß eingesetzt werden. Die Herstellung dieser Polymerisate wird in der deutschen Offenlegungsschrift 39 29 973 beschrieben.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Homopolymere von Diallylammoniumsalzen, Copolymere von Diallylammoniumsalzen und Acrylamiden, quaternierte Vinyl-pyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Ein bevorzugtes kationisches Polymer ist das Homopolymer aus dem Monomer mit der Formel (III). Diallyldimethylammonium-Homopolymere werden mit der INCI-Bezeichnung Polyquaternium-6 beispielsweise unter dem Handelsnamen Merquat® 100 (Nalco) vermarktet.

Die amphoteren bzw. kationischen Polymere sind in dem erfindungsgemäßen Mittel bevorzugt in einer Menge von 0,05 bis 20 Gew.-%, besonders bevorzugt von 0,1 bis 15 Gew.-% jeweils bezogen auf das Gewicht des anwendungsbereiten Mittels enthalten.

In einer bevorzugten Ausführungsform der vorliegenden Erfindung können die Mittel neben den erfindungswesentlichen Komponenten weiterhin mindestens ein ($C_8$ bis $C_{22}$)-Triacylglycerid enthalten.

Die ($C_8$ bis $C_{22}$)-Acylgruppen der Triacylglyceride leiten sich erfindungsgemäß bevorzugt von linearen oder verzweigten, gesättigten oder ungesättigten Fettsäuren ab. Typische Beispiele solcher Fettsäuren sind Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Arachidonsäure, Gadoleinsäure, Behensäure und Erucasäure sowie deren Mischungen. Die ($C_8$ bis $C_{22}$)-Triacylglyceride enthalten besonders bevorzugt mindestens eine ($C_8$ bis $C_{22}$)-Acylgruppe, die sich von einer ungesättigten Fettsäure ableitet, insbesondere von der Palmitoleinsäure, Ölsäure, Petroselinsäure, Elaeostearinsäure, Gadoleinsäure, Erucasäure, Linolensäure und/oder Linolsäure. Erfindungsgemäße Mittel, welche Palmitoleinsäure-haltige Triacylglyceride enthalten, sind besonders bevorzugt.

Die ($C_8$ bis $C_{22}$)-Triacylglyceride können synthetischer oder natürlicher Herkunft sein. Es ist erfindungsgemäß bevorzugt, als Triacylglyceridquelle ein natürliches Öl, bevorzugt Öle aus Nüssen wie z.B. Pekannußöl, Paranussöl, Kukuinußöl, Walnußöl, Haselnußöl, Erdnußöl oder Macadamianußöl, zu verwenden. Macadamianußöl ist ein besonders bevorzugtes natürliches Öl. Geeignete Macadamianußöle sind beispielsweise Öle, die bevorzugt aus dem Samenkern der Nüsse der *Macadamia tetraphylla, Macadamia integrifolia* sowie *Macadamia ternifolia* gewonnen werden. Dabei wird wiederum das Öl der *Macadamia ternifolia* bevorzugt als Triacylglyceridquelle zur Bereitstellung der erfindungsgemäßen Mittel verwendet. Das Triacylglycerid-Gemisch dieses Macadamianußöls weist beispielsweise eine Fettsäurezusammensetzung von:

| | |
|---|---|
| < 1 Gew.-% | Laurinsäure |
| < 1 Gew.-% | Myristinsäure |
| 8 bis 10 Gew.-% | Palmitinsäure |
| 16 bis 24 Gew.-% | Palmitoleinsäure |
| 2 bis 4 Gew.-% | Stearinsäure |
| 53 bis 67 Gew.-% | Ölsäure |
| 1,5 bis 4 Gew.-% | Linolsäure |
| < 1 Gew.-% | Linolensäure |
| 1,5 bis 3 Gew.-% | Arachinsäure |

(fortgesetzt)

| | |
|---|---|
| < 1 Gew.-% | Behensäure |
| < 1 Gew.-% | Erucasäure |

auf. Entsprechende Macadamianußöle werden beispielsweise unter der INCI-Bezeichnung Macadamia ternifolia von der Firma J.H. Müller vertrieben.

Die erfindungsgemäßen ($C_8$ bis $C_{22}$)-Triacylglyceride sind bevorzugt in einer Menge von 0,05 bis 10 Gew.-%, besonders bevorzugt von 0,1 bis 5 Gew.-%, jeweils bezogen auf das Gewicht des Mittels enthalten.

Das Mengenverhältnis der ($C_8$ bis $C_{22}$)-Triacylglyceride zur Verbindung mit der Formel (I) zu den amphoteren bzw. kationischen Polymeren beträgt bevorzugt 1 zu 1 zu 1 bis 1 zu 300 zu 300, besonders bevorzugt 1 zu 1 zu 1 bis 1 zu 100 zu 50, ganz besonders bevorzugt von 1 zu 2,5 zu 4 bis 1 zu 5 zu 5.

Eine ganz besonders bevorzugte Ausführungsform der erfindungsgemäßen Wirkstoffkombination ist die Kombination einer Polyammonium-Polysiloxan-Verbindung, einer Verbindung gemäß Formel (I) und einem Homopolymer aus dem Monomer mit der Formel (III).

Das erfindungsgemäße Mittel kann als Lotion oder Creme, d.h. als entsprechende W/O-Emulsion bzw. O/W-Emulsion sowie als Gel vorliegen. Bevorzugt liegt das erfindungsgemäße Mittel in der Ausführungsform als Färbemittel als Creme oder (insbesondere klares) Gel vor.

Der Begriff "Creme" betrifft im Sinne der Erfindung W/O-Emulsionen bzw. O/W-Emulsionen von Fetten und/ oder Fettalkoholen und Wasser. Dabei können in der Wasserphase auch organische Lösemittel wie niedermolekulare Monoalkohole und/oder Polyole (Definition siehe unten) enthalten sein. Solche Cremes werden durch das Emulgieren von beispielsweise festen und/oder flüssigen Paraffinen, Wollwachs, Bienenwachs, synthetischen Wachsestern, Fettalkoholen, Sterolen oder Sterolestern erhalten. Bevorzugte Vertreter dieser Stoffe werden weiter unten definiert. Als Emulgatoren dienen bevorzugt nichtionogene Tenside aus mindestens einer der folgenden Gruppen:

(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) $C_{12/18}$Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologen-gemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus DE-PS 20 24 051 als Rückfettungsmittel für kosmetische Zubereitungen bekannt.

$C_{8/18}$-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für

solche technischen Produkte übliche Homologenverteilung zugrunde liegt.

Der Begriff "Gel" wird in "Faraday discussions of the chemical society, No. 57, 1974 (Gels and Gelling Processes), P.J. Flory, Seite 7-18", näher charakterisiert. Demnach werden Gele in 4 Untergruppen eingeteilt:

1. Gele mit lamellaren Ordnungsstrukturen einschließlich Gel-Mesophasen, z.B. Tensidgele, insbesondere Seifengele.
2. Gele, die aus polymeren, dreidimensionalen, kovalent gebundenen Netzwerken, die ohne Zerstörung der Kovalenzbindungen in Lösungsmitteln unlöslich sind, aufgebaut sind.
3. Gele aus polymeren Netzwerken mit lokalen Ordnungsstrukturen, die durch physikalische Kräfte zusammengehalten werden, z.B. Gelatine, Homo- und Copolymere von Acryl- und Methacrylsäure, Alginate, Carrageenan.
4. Gele gebildet aus feinverteilten Bestandteilen, z.B. Niederschlägen, die i.a. eine große geometrische Anisotropie aufweisen, z.B. $V_2O_5$-Gele oder Gele, die sich durch Aggregation von Proteinen bilden.

Gele im Sinne dieser Ausführungsform der Erfindung sind solche, die unter Punkt 1. und Punkt 3. der obigen Auflistung fallen. Als "Gel" im Sinne der vorliegenden Erfindung sind unter anderem Transparentgele wie sie z.B. in den US-Patentschriften US 3 101 300 und US 3 101 301 beschrieben werden, zu verstehen. Diese Transparentgele werden als Mikroemulsionsgele bezeichnet. Eine allgemeingültige Definition für Mikroemulsionsgele ist in "Happi: Household Pers. Prod. Ind. 30 (1993), Nr. 2, p. 58-64" angegeben.

Ein Gel ist als klar zu bewerten, wenn bei einer Schichtdicke von 1 cm die Extinktion bei einer Wellenlänge, in dem die gelförmige Zusammensetzung keine Absorptionsbanden aufweist (typischerweise bei einer Wellenlänge von 800 nm) höchstens 0.05 Extinktionseinheiten beträgt.

Das Gel sollte vorzugsweise fließfähig sein, und durch eine hohe Strukturviskosität gekennzeichnet sein, d.h., diese Zubereitungen erscheinen unter dem Einfluss geringer Scherkräfte, etwa des Eigengewichts, relativ hochviskos, lassen sich aber bei Anwendung höherer Scherkräfte, z.B. beim Verrühren sehr leicht bewegen.

Es ist erfindungsgemäß bevorzugt, als klare, wässrige Gelgrundlage eine Zusammensetzung, enthaltend

a) 0,5 bis 10 Gew.-% eines Anlagerungsproduktes von 1 bis 5 Mol Ethylenoxid an einen linearen Fettalkohol mit 8 bis 22 C-Atomen,
b) 0,1 bis 15 Gew.-% eines gesättigten oder ungesättigten, linearen oder verzweigten Alkohols mit 8 bis 36 C-Atomen und
c) 0,1 bis 15 Gew.-% einer flüssigen Fettsäure mit 16 bis 22 C-Atomen in Form einer wasserlöslichen Seife

zu verwenden. Dieses Transparentgel eignet sich besonders gut zur Einarbeitung der erfindungsgemäßen Wirkstoffkombination.

Erfindungsgemäß verwendbare Anlagerungsprodukte von 1 bis 5 Mol Ethylenoxid an einen linearen Fettalkohol mit 8 bis 22 C-Atomen werden beispielsweise mit der INCI-Bezeichnung Laureth-2 (Handelsprodukt Dehydol® LS 2 Deo N der Fa. Cognis), Laureth-3 (Handelsprodukt Dehydol® LS 3 der Fa. Cognis), Laureth-4 (Handelsprodukt Dehydol® LS 4 der Fa. Cognis), Ceteareth-2 (Lowenol® C 279 der Fa. Lowenstein), Steareth-2 (Eumulgin® S 2 der Fa. Cognis) bzw. Steareth-4 (Arlypon® SA 4 der Fa. Cognis) bekannt.

Bei den erfindungsgemäß einzusetzenden gesättigten oder ungesättigten, linearen oder verzweigten Alkoholen mit 8 bis 36 C-Atomen handelt es sich vorzugsweise um Fettalkohole und/oder Guerbetalkohole.

Unter Fettalkoholen sind im Sinne der Erfindung primäre aliphatische Alkohole der Formel (V) zu verstehen,

$$R^{10}OH \qquad (V)$$

in der $R^{10}$ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind technische Fettalkoholmischungen mit 12 bis 18 Kohlenstoffatomen wie beispielsweise Kokos-, Palm-, Palmkern- oder Talgfettalkohol, insbesondere Kokos- und/ oder Talgfettalkohol.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20):

Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Die zur Seifenbildung geeigneten Fettsäuren sind bevorzugt flüssige oder niedrigschmelzende ungesättigte lineare $C_{16}$-$C_{22}$-Fettsäuren wie Palmitoleinsäure, Ölsäure, Elaidinsäure, Myristinsäure, Petroselinsäure, Petroselaidinsäure, Gadoleinsäure, Erucasäure, Brassidinsäure sowie Gemische dieser Fettsäuren untereinander und gegebenenfalls mit geringeren Anteilen gesättigter linearer Fettsäuren mit 12 bis 22 C-Atomen. Andere ebenfalls geeignete Fettsäuren sind verzweigte Fettsäuren mit 16 bis 22 C-Atomen, z.B. die 2-Hexyldecansäure, Isostearinsäure und die 2-Octyldodecansäure.

Zur Überführung der Fettsäuren in wasserlösliche Seifen eignen sich Alkalihydroxide und Alkalicarbonate, Ammoniak, Mono-, Di- und Trialkanolamine mit 2 bis 4 C-Atomen in der Alkanolgruppe sowie alkalische Aminosäuren wie beispielsweise Arginin, Ornithin, Lysin und/ oder Histidin.

Es ist erfindungsgemäß bevorzugt, wenn das erfindungsgemäße Konditioniermittel zusätzlich, wiederum bevorzugt zusammen mit den drei vorgenannten Komponenten a) bis c), 0,5 bis 10 Gew.-% eines Anlagerungsproduktes von 15 bis 100 Einheiten Ethylenoxid, insbesondere von 15 bis 50 Mol Ethylenoxid jeweils pro Molekül eines linearen Fettalkohols mit 8 bis 22 C-Atomen, in einer wasserhaltigen Zusammensetzung enthält. Es handelt sich dabei ganz besonders bevorzugt um Ceteareth-15 oder Ceteareth-50, welche als Eumulgin® CS 15 bzw. Eumulgin® CS 50 von der Firma Cognis Deutschland GmbH vermarktet werden.

Als Anlagerungsprodukte von 1 bis 4 bzw. 15 bis 100 Mol Ethylenoxid an einen linearen Fettalkohol mit 12 bis 22 C-Atomen eignen sich alle nach den bekannten technischen Oxethylierungsverfahren erhältlichen Addukte. Bevorzugt sind die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind.

Neben den Transparentgelen können erfindungsgemäß aber auch nicht klare, d.h. trübe beziehungsweise milchige, Gele bevorzugt sein.

Die erfindungsgemäßen Mittel sind wasserhaltige Zusammensetzungen. Darunter fallen auch wässrig-alkoholische Systeme. Unter wässrig-alkoholisch sind im Sinne der vorliegenden Erfindung wasserhaltige Zusammensetzungen mit einem Gehalt von höchstens 70 Gew.-% eines niedermolekularen Monoalkohols und/oder eines Polyols zu verstehen.

Unter niedermolekularen Monoalkoholen sind im Sinne der vorliegenden Erfindung mit Wasser mischbare Alkohole mit 1 bis 5 C-Atomen zu verstehen. Diese Monoalkohole tragen nur eine Hydroxygruppe. Vorzugsweise handelt es sich bei den niedermolekularen Monoalkoholen um Ethanol, Propanol und/ oder Isopropanol.

Polyole tragen im Sinne der Erfindung mindestens zwei Hydroxygruppen, bevorzugt zwei bis vier Hydroxygruppen. Als Polyole sind insbesondere solche mit 2 bis 6 C-Atomen bevorzugt. Es eignen sich z.B. Ethylenglycol, 1,2-Propylenglycol, 1,3-Propylenglycol, Glycerin, Erythrit, Trimethylolpropan, Diethylenglycol und Dipropylenglycol. 1,2-Propylenglycol ist besonders bevorzugt.

Bevorzugt enthalten die erfindungsgemäßen Mittel mindestens einen niedermolekularen Monoalkohol und mindestens ein Polyol.

Als wasserlösliche synthetische Tenside eignen sich bevorzugt anionische, amphotere, zwitterionische und nichtionische Tenside mit guter Wasserlöslichkeit und mit gutem Kalkseifendispergiervermögen. Solche Tenside weisen in der Regel eine lipophile lineare Alkyl- oder Acylgruppe mit 12 bis 18 C-Atomen und eine stark dissoziierte ionische Gruppe oder eine nichtionische wasserlöslichmachende Polyethergruppe auf. Geeignet sind z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen. Weitere geeignete Aniontenside sind z.B. lineare Alkansulfonate und $\alpha$-Olefinsulfonate mit 12 bis 18 C-Atomen. Geeignete nichtionogene Tenside sind z.B. die Anlagerungsprodukte von 7 bis 30 Mol Ethylenoxid an lineare Fettalkohole mit 12 bis 18 C-Atomen, an Fettsäuren mit 12 bis 18 C-Atomen sowie an Fettsäuremonoglyceride und an Fettsäuresorbitanmonoester. Bevorzugt geeignete nichtionogene Tenside sind die Fettalkylaminoxide und insbesondere die Fettalkylglykoside, vorzugsweise Fettalkylglucoside. Die Fettalkylgruppe kann in den genannten Produkten 12 bis 18 C-Atome aufweisen. Besonders bevorzugt im Sinne der vorliegenden Erfindung sind auch amphotere Tenside, wie beispielsweise N-Fettalkyl-dimethyl-glycin oder N-Fettalkylaminopropionsäure und/ oder zwitterionische Tenside, z.B. N-Fettalkyldimethylammoniumglycinat oder N-Fettacylaminopropyldimethylglycinat. Diese amphoteren Tenside können zusätzlich zu den Verbindungen gemäß Formel (I) in den erfindungsgemäßen Mitteln enthalten sein und sind von den Verbindungen der Formel (I) verschieden. Weiterhin bevorzugt werden Kationtenside wie quartäre Ammoniumverbindungen (QAV) insbesondere quaternierte Trialkylammoniumverbindungen mit Alkylresten einer Kettenlänge von $C_8$ bis $C_{22}$.

Es hat sich gezeigt, dass durch den Zusatz weiterer, begrenzt wasserltislicher, nichtionogener Tenside eine

Verdickung der erfindungsgemäßen Mittel, insbesondere der Gele, erreicht wird.

Zusätzlich können die erfindungsgemäßen Mittel Anlagerungsprodukte von 1 bis 4 Mol Ethylenoxid an ein Fettalkylamin enthalten. Als Anlagerungsprodukte von 1 bis 4 Mol Ethylenoxid an ein lineares Fettalkylamin mit 12 bis 22 C-Atomen eignen sich alle nach bekannten technischen Verfahren zugänglichen Addukte, die auch im Handel erhältlich sind. Besonders geeignet ist das Anlagerungsprodukt von 2 Mol Ethylenoxid an ein $C_{12}$-$C_{18}$-Kokosalkylamin.

Geeignete Verbindungen der Formel (VI)

$$R^{11}\text{-}(OC_2H_4)_x\text{-}A\text{-}(C_2H_4O)_y\text{-}R^{12} \qquad \text{(VI)}$$

in welcher A ein Sauerstoffatom ist, sind Dialkylether mit 12 bis 18 C-Atomen in den Alkylgruppen $R^{11}$ und $R^{12}$. Solche Produkte sind literaturbekannt. Noch besser geeignet sind die Produkte der Formel (VI), in welchen x oder y oder beide = 1 sind. Solche Dialkyloxethylether lassen sich durch literaturbekannte Veretherungsverfahren aus Fettalkoholen und Fettalkyloxyethanolen herstellen. Die Produkte der Formel (VI), in welchen A eine Gruppe

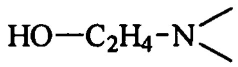

ist, lassen sich z.B. aus Triethanolamin durch O-Alkylierung mit 2 Mol eines Schwefelsäurehalbestersalzes eines ($C_{12}$ bis $C_{22}$)-Fettalkohols nach dem in DE 35 04 242 beschriebenen Verfahren zur Herstellung von Etheraminen gewinnen.

Besonders bevorzugte Verbindungen der Formel (VI) sind z.B. Dicetylstearylether, Dicetylstearyldioxyethylether und N,N-Bis-(2-cetyl-/stearyl-oxyethyl)-aminoethanol.

Während mit Anlagerungsprodukten von 1 bis 5 Mol Ethylenoxid an einen linearen Fettalkohol die erforderliche Verdickung meist allein durch diese Komponente erreicht wird, kann es bei Einsatz der Anlagerungsprodukte von 1 bis 4 Mol Ethylenoxid an ein lineares Fettalkylamin vorteilhaft sein, diese in Kombination mit 1 bis 10 Gew.-% einer Verbindung der Formel (VI) zu verwenden.

Desweiteren enthält das erfindungsgemäße Mittel bevorzugt zusätzlich mindestens ein anionisches Acrylsäure- und/oder Methacrylsäure-Polymerisat oder -Copolymerisat. Bevorzugte Polymerisate dieser Art sind:

- Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren, wie sie z.B. in GB 870 994 beschrieben sind .
- Mischpolymerisate (aus DE 11 64 095) aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll® D (BASF).
- die in DE 34 45 549 beschriebenen Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Diese anionischen Polymerisate fallen erfindungsgemäß nicht unter die Definition der erfindungsgemäßen amphoteren Polymere.

Die anionischen Acrylsäure- und/oder Methacrylsäure-Polymerisate oder -Copolymerisate sind in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten.

Insbesondere bevorzugt sind in den erfindungsgemäßen Mitteln zusätzlich Proteine und/ oder Proteinderivate pflanzlicher oder tierischer Herkunft wie beispielsweise Erbsen-, Soja-, Weizen- und Mandelproteinhydrolysat oder Akazienprotein sowie Collagen- oder Keratinhydrolysat enthalten. Die erfindungsgemäßen Mittel enthalten die Proteine und/oder Proteinderivate pflanzlicher oder tierischer Herkunft bevorzugt in einer Menge von 0,05 bis 10 Gew.-%, insbesondere 0,1 bis 3 Gew.-%, jeweils bezogen auf das Gewicht des gesamten Mittels.

Weiterhin ist es bevorzugt, in die erfindungsgemäßen Mittel zusätzlich mindestens ein Pflanzenextrakt einzuarbeiten.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Bevorzugte Pflanzenextrakte sind beispielsweise Extrakte von Blüten (Lindenblüten, Kamillen, Lilie, Lavendel, Rosen, Jasmin, Neroli, Ylang-Ylang), Stengeln und Blättern (Cassis, Rosskastanie, Rooibos, Birke, Melisse, Klee, Weinblättern, Geranium, Patchouli, Petitgrain), Früchten (Anis, Cassis, Koriander, Kümmel, Wacholder), Fruchtschalen (Bergamotte, Zitrone, Orangen), Wurzeln (Macis, Angelica, Sellerie, Kardamon, Costus, Iris, Calmus, Ginseng), Hölzern (Pinien-, Sandel-, Guajak-, Zedern-, Rosenholz), Kräutern und Gräsern (Estragon, Lemongras, Salbei, Thymian, Rosmarin), Nadeln und Zweigen (Fichte, Tanne, Kiefer, Latschen, Sandelholz), Harzen und Balsamen (Galbanum, Elemi, Benzoe, Myrrhe,

Olibanum, Opoponax).

Besonders bevorzugt werden die Pflanzenextrakte ausgewählt aus mindestens einem Extrakt aus der Gruppe Hamamelis (Hamamelis virginiana L.), Weinblättern (Vitis vinifera L.), Rosen (Rosa gallica L.), Sandelholz (Pterocarpus Santalinus), Rooibos (Aspalathus linearis), Rosskastanie (Aesculus Hippocastanum L.), Klee (insbesondere Rotklee, Trifolium pratense), Zimt (Cinnamomum zeylanicum nees) und Cassis (insbesondere aus Cassis Blättern, Ribes nigrum L.).

Solche bevorzugt verwendeten Extrakte werden unter den Bezeichnungen Herbasol® von der Firma Cosmetochem oder Extrapon® von der Firma Symrise vermarktet.

Die Pflanzenextrakte sind in dem erfindungsgemäßen Mittel bevorzugt in einer Menge von 0,05 bis 5 Gew.-%, insbesondere 0,1 bis 2 Gew.-%, jeweils bezogen auf das Gewicht des gesamten Mittels, enthalten.

Weiterhin kann das erfindungsgemäße Mittel zusätzlich mindestens ein Saccharid als Mono-, Di-, Oligo- und/ oder Polysaccharid enthalten. Bevorzugt sind hierbei Mono-, Di- und/oder Oligosaccharide, wie insbesondere D-Glucose, D-Galactose, D-Mannose, D-Fructose, L-Arabinose, D-Xylose, D-Ribose u. 2-Desoxy-D-ribose, Saccharose, Maltose, Lactose, Galactose, Trehalose, Cellobiose, Gentiobiose und Isomaltose.

Besonders bevorzugt ist eine Kombination aus D-Glucose und D-Fructose. D-Glucose und D-Fructose können dem erfindungsgemäßen Mittel jeweils in ihrer isolierten Form oder z.B. als Honig bzw. Honigextrakt zugesetzt werden.

Das erfindungsgemäße Mittel kann insbesondere als 2-in-1-Färbemittel konfektioniert sein. In dieser Ausführungsform der Erfindung enthält es zusätzlich

- als Oxidationsfarbstoffvorprodukt mindestens eine Entwicklerkomponente und gegebenenfalls mindestens eine Kupplerkomponente und/oder
- mindestens einen direktziehenden Farbstoff.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1)

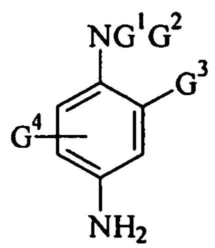

(E1)

wobei

- $G^1$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $G^2$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$- Mesylaminoalkoxyrest oder einen $C_1$- bis $C_4$-Carbamoylaminoalkoxyrest;
- $G^4$ steht für ein Wasserstoffatom, ein Halogenatom oder einen $C_1$- bis $C_4$-Alkylrest oder
- wenn $G^3$ und $G^4$ in ortho-Stellung zueinander stehen, können sie gemeinsam eine verbrückende $\alpha,\omega$-Alkylendioxygruppe, wie beispielsweise eine Ethylendioxygruppe bilden.

Beispiele für die als Substituenten in den erfindungsgemäßen Verbindungen genannten $C_1$- bis $C_4$-Alkylreste

sind die Gruppen Methyl, Ethyl, Propyl, Isopropyl und Butyl. Ethyl und Methyl sind bevorzugte Alkylreste. Erfindungsgemäß bevorzugte $C_1$- bis $C_4$-Alkoxyreste sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte $C_2$- bis $C_4$-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, $C_1$- bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(a,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl] amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

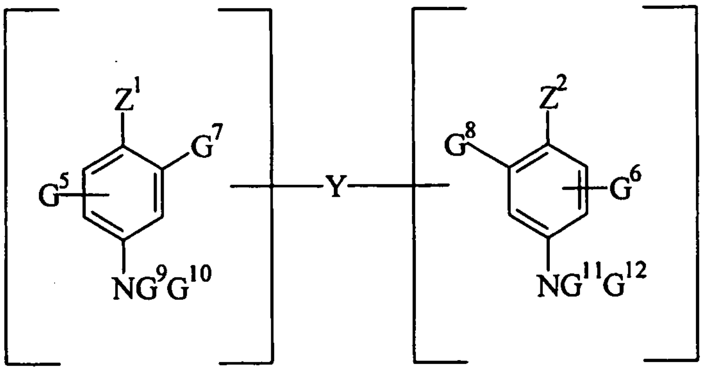

(E2)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/ oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein kann, oder eine direkte Bindung,
- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$- Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur

Verbrückung Y oder einen $C_1$- bis $C_4$-Alkylrest,

mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3)

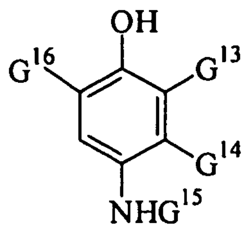

(E3)

wobei:

- $G^{13}$ steht für ein Wasserstoffatom, ein Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest, einen Hydroxy-($C_1$- bis $C_4$)-alkylaminorest, einen $C_1$-bis $C_4$-Hydroxyalkoxyrest, einen $C_1$- bis $C_4$-Hydroxyalkyl-($C_1$-bis $C_4$)-aminoalkylrest oder einen (Di-$C_1$- bis $C_4$-Alkylamino)-($C_1$- bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest oder einen $C_1$- bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxypyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht:

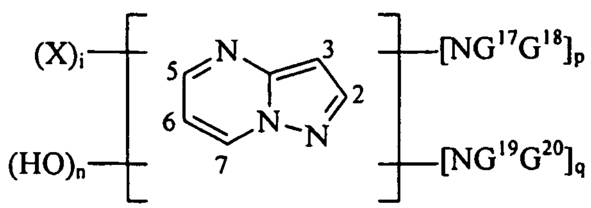

(E4)

wobei:

- $G^{17}$, $G^{18}$, $G^{19}$ und $G^{20}$ unabhängig voneinander stehen für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest einen ($C_1$- bis $C_4$)-Alkoxy-($C_1$- bis $C_4$)-alkylrest, einen $C_1$- bis $C_4$-Aminoalkylrest, der gegebenenfalls durch ein Acetyl-Ureid- oder einen Sulfonyl-Rest geschützt sein kann, einen ($C_1$- bis $C_4$)-Alkylamino-($C_1$- bis $C_4$)-alkylrest, einen Di-[($C_1$- bis $C_4$)-alkyl]-($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$-bis $C_4$)-[Hydroxyalkyl]-($C_1$- bis $C_4$)-aminoalkylrest,
- die X-Reste stehen unabhängig voneinander für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest, einen $C_1$- bis $C_4$-Aminoalkylrest, einen ($C_1$- bis $C_4$)-Alkylamino-($C_1$- bis $C_4$)-alkylrest, einen Di-[($C_1$- bis $C_4$)alkyl]- ($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$-hydroxyalkyl)-aminoalkylrest, einen Aminorest, einen $C_1$- bis $C_4$-Alkyl- oder Di-($C_1$- bis $C_4$-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
  mit der Maßgabe, dass
- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen

definiert.

Wenn das Pyrazolo[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

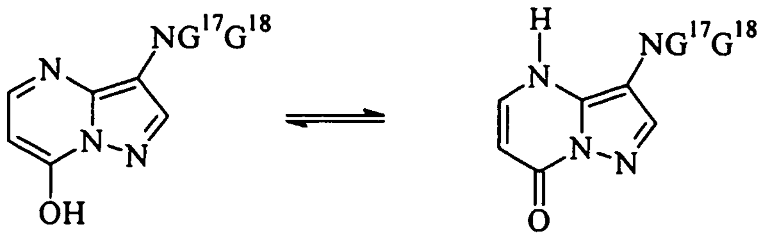

Unter den Pyrazolo[1,5-a]-pyrimidinen der obenstehenden Formel (E4) kann man insbesondere nennen:

- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
  sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (VIIa),

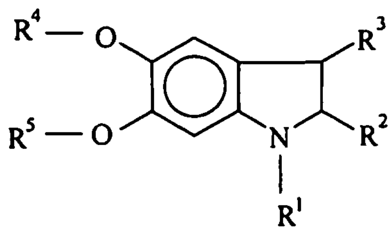

(VIIa)

in der unabhängig voneinander

- $R^1$ steht für Wasserstoff, eine $C_1$-$C_4$-Alkylgruppe oder eine $C_1$-$C_4$-Hydroxy-alkylgruppe,

- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,
  sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (VIIb),

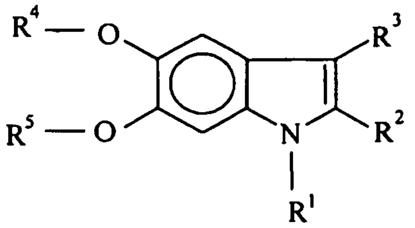

(VIIb)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,

- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino) ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol

sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Bevorzugte direktziehende Farbstoffe, die in den erfindungsgemäßen Mitteln Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908 in den Ansprüchen 6 bis 11 genannt werden.

Bevorzugte kationische direktziehende Farbstoffe der Gruppe (c) sind insbesondere die folgenden Verbindungen:

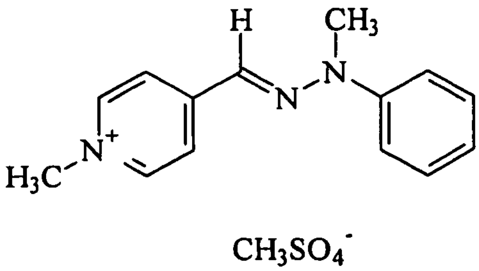

(DZ1)

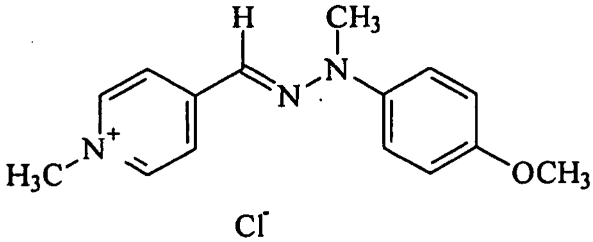

(DZ2)

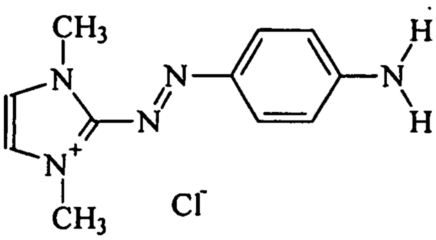

(DZ3)

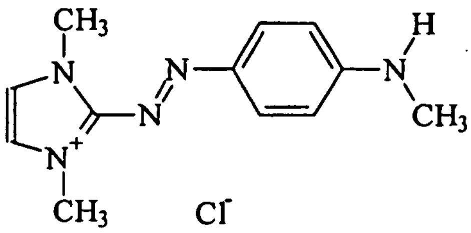

(DZ4)

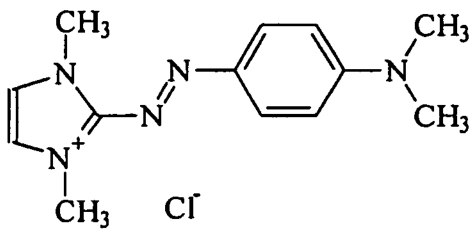

(DZ5)

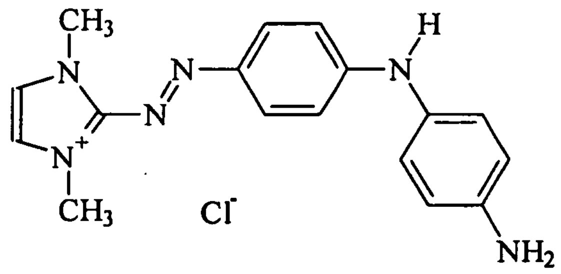

(DZ6)

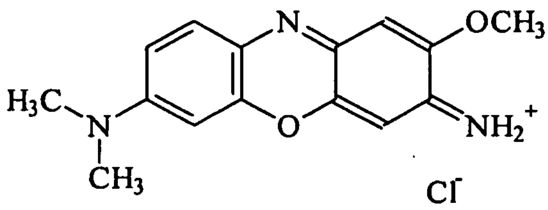

(DZ7)

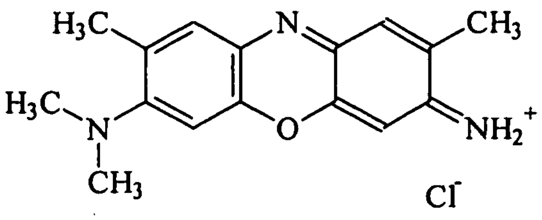

(DZ8)

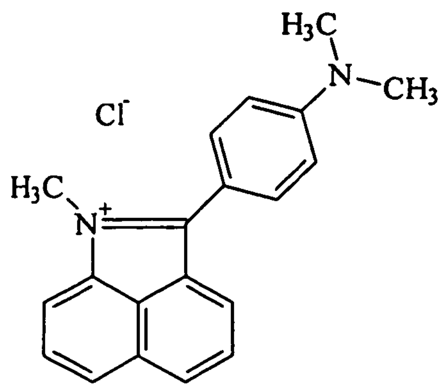

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Zusammensetzungen gemäß dieser Ausführungsform enthalten die direktziehenden Farbstoffe bevorzugt in einer Menge von 0,01 bis 20 Gew.-%, bezogen auf das gesamte Mittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen,

ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Wenn das erfindungsgemäße Mittel Oxidationsfarbstoffvorprodukte enthält, kann die eigentliche oxidative Färbung der Fasern grundsätzlich mit Luftsauerstoff erfolgen. Bevorzugt wird jedoch ein chemisches Oxidationsmittel eingesetzt, besonders dann, wenn neben der Färbung mit den Oxidationsfarbstoffvorprodukten und/oder direktziehenden Farbstoffen ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie an Natriumborat in Frage.

Soll das erfindungsgemäße Mittel als Kombinationspräparat in Form eines Bleich- und Konditioniermittels bereitgestellt werden, enthält das anwendungsbereite Mittel insbesondere eine Mischung aus Persulfaten und Wasserstoffperoxid als Oxidationsmittel.

Soll das erfindungsgemäße Mittel als Kombinationspräparat in Form eines oxidativen Haarfärbe- und Konditioniermittels bereitgestellt werden, besteht es bevorzugt aus zwei Komponenten, nämlich einer Zubereitung, enthaltend die Farbstoffvorprodukte bzw. die direktziehenden Farbstoffe (die im weiteren als Farbstoffträger bezeichnet wird) und einer Oxidationsmittelzusammensetzung.

Ein zweiter Gegenstand der Erfindung ist daher ein Mittel, welches unmittelbar vor der Anwendung aus zwei Komponenten gemischt wird, wobei die erste Komponente als Farbstoffträger ein Mittel des ersten Erfindungsgegenstandes, enthaltend zusätzlich

- mindestens eine Entwicklerkomponente und gegebenenfalls mindestens eine Kupplerkomponente und/oder
- mindestens einen direktziehenden Farbstoff

bedeutet und die zweite Komponente eine Oxidationsmittelzusammensetzung, enthaltend mindestens ein chemisches Oxidationsmittel, insbesondere Wasserstoffperoxid, ist.

Optional kann das erfindungsgemäße Mittel auch zusammen mit einem Oxidationsaktivator auf das Haar aufgebracht werden, der die Oxidation der Farbstoffvorprodukte durch das Oxidationsmittel, aktiviert. Als Oxidationsmittel dient, wie zuvor erwähnt, Luftsauerstoff oder zusätzliche chemische Oxidationsmittel. Die Oxidationsaktivatoren werden bevorzugt ausgewählt aus der Gruppe, die gebildet wird aus Carbonaten, Hydrogencarbonaten, Carbamaten, Carbonsäureestern oder deren Salze, Aldehyden, insbesondere aliphatischen Aldehyden, 1,3-Dihydroxyaceton, Imidazol und seinen Derivaten, Alkali- und Ammoniumperoxidisulfaten, Metallionen, Iodiden, Chinonen und Enzymen.

Die Oxidationsaktivatoren sind bevorzugt in Mengen von 0.01 bis 5 Gew.%, bezogen auf das Gewicht des gesamten Mittels, in den erfindungsgemäßen Mitteln enthalten.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche (oxidative) Haarfärbemittel wird zweckmäßigerweise unmittelbar vor der Anwendung durch

Mischung der Oxidationsmittelzusammensetzung mit dem Farbstoffträger, bevorzugt im Gewichtsverhältnisbereich von 1 zu 4 bis 4 zu 1, insbesondere von 1 zu 2 bis 2 zu 1, hergestellt. Das dabei entstehende gebrauchsfertige Haarfärbepräparat sollte bevorzugt einen pH-Wert im Bereich von 6 bis 12, insbesondere von pH 7 bis 11, aufweisen. Besonders bevorzugt ist die Anwendung der Haarfärbemittel in einem schwach alkalischen Milieu.

Es wird angestrebt, dass der (insbesondere gelförmige und klare) Farbstoffträger fließfähig ist und bevorzugt eine Viskosität von höchstens 100 mPa·s (20 °C), bevorzugt von höchstens 20 mPa·s (20 °C) besitzt. Wird diese Zusammensetzung mit einer Oxidationsmittelzusammensetzung vermischt, weist der anwendungsfertige (insbesondere gelförmige und klare) Färbeansatz bevorzugt eine Viskosität von wenigstens 3 Pa·s (20 °C), bevorzugt von 8 bis 20 Pa·s (20 °C), auf (Viskositätsmessungen jeweils mit dem Rotationsviskosimeter Brookfield, Typ RTV, Spindel 4, 4 rpm).

In einer weiteren Ausführungsform der vorliegenden Erfindung können tubenfähige Gele bevorzugt sein, die Viskositäten in der Größenordnung einer herkömmlichen Emulsion aufweisen. Die Viskositäten der Gele dieser Ausführungsform liegen bevorzugt in einem Bereich von 10 000 bis 100 000mPas, besonders bevorzugt in einem Bereich von 15 000 bis 50 000 mPas, ganz besonders bevorzugt in einem Bereich von 20 000 bis 35 000mPas (Viskositätsmessungen jeweils mit dem Rotationsviskosimeter Brookfield, Typ RTV, Spindel 4, 4 rpm bei 20°C). Derartige Gele werden insbesondere dann erhalten, wenn der Gehalt an niedermolekularen Monoalkoholen und/oder Polyolen deutlich abgesenkt wird beziehungsweise ganz auf diese Komponenten verzichtet wird. Im Rahmen dieser Ausführungsform ist ein Gehalt an niedermolekularen Monoalkoholen und/oder Polyolen von weniger als 50Gew.-%, vorzugsweise weniger als 30Gew.-%, ganz vorzugsweise weniger als 10 Gew.-%, erfindungsgemäß bevorzugt. Im Rahmen dieser Ausführungform kann es ganz besonders bevorzugt sein, trübe Gele der genannten Viskosität einzusetzen.

Als erfindungsgemäß bevorzugte Oxidationsmittelzusammensetzung für die Ausführungsform als Färbemittel eignet sich hervorragend eine wässrige Zusammensetzung, enthaltend

| | |
|---|---|
| 3 bis 12 Gew.-% | Wasserstoffperoxid |
| 0,1 bis 5 Gew.-% | eines wasserlöslichen, synthetischen Tensids und |
| 1 bis 5 Gew.-% | eines dispergierten Acrylsäure- und/oder Methacrylsäure-Polymerisats oder -Copolymerisats. |

Sie kann aber auch ohne Wasserstoffperoxid oder im einfachsten Fall allein aus Wasser bestehen, so dass die Oxidation von Oxidationsfarbstoffvorprodukten durch den Luftsauerstoff herbeigeführt wird.

Als wasserlösliche synthetische Tenside können in der Oxidationsmittelzusammensetzung die zuvor für das erfindungsgemäße Mittel genannten optionalen Tenside aus anionischen, amphoteren, zwitterionischen und nichtionischen Tensiden oder Gemischen davon verwendet werden. Bevorzugt werden anionische Tenside, z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen in Form ihrer Alkali-, Magnesium-, Ammonium- oder Alkanolammoniumsalze eingesetzt.

Die Oxidationsmittelzusammensetzung enthält außerdem bevorzugt Komplexbildner und Puffersalze zur Einstellung eines pH-Wertes von 2 bis 5. In diesem schwach sauren Medium bleiben die Acrylsäure- und/oder Methacrylsäure-Polymerisat-Dispersionen dünnflüssig und stabil. Beim Vermischen mit des alkalischen Farbstoffträgers, der Ammoniak und Puffersalze zur Einstellung eines pH-Wertes von 8 bis 10 enthält, steigt der pH-Wert der Mischung an und die Carboxylgruppen des Polymerisats oder Copolymerisats werden in die Salzform überführt. Dabei beginnen die Polymerisate sich im wässrigen Medium zu lösen und die Viskosität der Lösung anzuheben.

Besonders günstig für den Viskositätsaufbau nach dem Vermischen ist der Gehalt an dispergierten Acrylsäure- und/oder Methacrylsäure-Polymerisat oder -Copolymerisat in der Oxidationsmittelzusammensetzung. Bevorzugte Dispersionen von entsprechenden Polymerisaten sind solche, welche die zuvor genannten bevorzugten Acrylsäure- und/oder Methycrylsäre-Polymerisate oder -Copolymerisate enthalten.

Die erfindungsgemäßen Mittel können zusätzlich alle auf diesen Gebieten bekannten und üblicherweise verwendeten Wirk- und Hilfsstoffe enthalten.

Ein dritter Gegenstand der vorliegenden Erfindung ist ein Verfahren zur Behandlung keratinhaltiger Fasern, bei dem ein erfindungsgemäßes Mittel auf die Fasern aufgetragen wird und nach einer Einwirkzeit wieder abgespült wird. Die Einwirkungszeit beträgt bevorzugt 1 bis 20 Minuten.

Eine bevorzugte Ausführungsform des erfindungsgemäßen Verfahrens ist ein Verfahren zur Färbung und Konditionierung keratinischer Fasern, bei dem das erfindungsgemäße Mittel des ersten Gegenstandes, enthaltend zusätzlich

- mindestens eine Entwicklerkomponente und gegebenenfalls mindestens eine Kupplerkomponente und/oder
- mindestens einen direktziehenden Farbstoff

auf die Fasern aufgetragen wird und nach einer Einwirkzeit wieder abgespült wird.

Die Anwendungstemperaturen dieser Ausführungsform können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein weiterer Gegenstand ist eine Verpackungseinheit (Kit), welche mindestens ein getrennt konfektioniertes, erfindungsgemäßes Mittel des ersten Erfindungsgegenstandes enthält. Zusätzlich kann das Kit mindestens eine getrennt konfektionierte Oxidationsmittelzusammensetzung enthalten, insbesondere dann, wenn ein (oxidatives) Färbemittel bereitgestellt werden soll. Darüber hinaus kann das Kit zusätzlich optional eine Gebrauchsanweisung, Applikationshilfen oder Schutzhandschuhe enthalten.

Eine Verpackungseinheit bildet sich erfindungsgemäß, wenn getrennt konfektionierte Zusammensetzungen durch eine gemeinsame Umverpackung, wie z.B. eine Faltschachtel oder eine Banderole, zusammengeführt werden. Im Sinne der Erfindung gilt es ebenso als Verpackungseinheit, wenn eine gemeinsame oder sequenzielle Benutzung getrennt konfektionierter Zusammensetzungen durch Anweisung in der Gebrauchsanleitung beziehungsweise in der Werbung vorgeschlagen wird.

## Beispiele

*1 Klare Gele*

1.1 Ausfärbung

Es wurden folgende klare, gelförmige Farbstoffträger nach einem herkömmlichen Verfahren hergestellt (sofern keine anders lautenden Angaben gemacht werden, verstehen sich die Mengenangaben in Gew.-%):

Tabelle 1:

| Rohstoff | V1 | E1 |
|---|---|---|
| Dehydol® LS 2 deo N | 4,90 | 4,90 |
| Eumulgin CS 50 | 5,00 | 5,00 |
| Lorol techn.® | 8,00 | 8,00 |
| Edenor® PK1805 | 6,75 | 6,75 |
| Isopropanol | 14,50 | 14,50 |
| 1,2-Propylenglykol | 6,75 | 6,75 |
| Texapon® NSO | 4,50 | 4,50 |
| Monoethanolamin | 5,00 | 5,00 |
| L-Arginin | 1,00 | 1,00 |
| Natriumsulfit | 0,10 | 0,10 |
| Ascorbinsäure | 0,40 | 0,40 |
| Turpinal® SL | 0,20 | 0,20 |
| Parfüm | 0,60 | 0,60 |
| Merquat® 280 | 1,50 | 1,50 |
| Miranol® C2M SF | 1,00 | 1,00 |
| Macadamianußöl | 0,40 | 0,40 |
| Herbasol Komplex SC | 0,30 | 0,30 |
| Erfindungsgemäßes Silikon | -- | 2,00 |
| p-Toluylendiamin, freie Base 25% | 1,93 | 1,93 |
| 3-Methyl-4-aminophenol | 0,50 | 0,50 |
| Resorcin | 0,57 | 0,57 |

(fortgesetzt)

| Rohstoff | V1 | E1 |
|---|---|---|
| m-Aminophenol | 0,05 | 0,05 |
| 5-Amino-2-methylphenol | 0,15 | 0,15 |
| 6-Chlor-4-nitro-2-aminophenol | 0,07 | 0,07 |
| Wasser | ad 100 | ad 100 |

Unmittelbar vor der Anwendung am Haar wurden die Farbstoffträger gemäß Tabelle 1 jeweils mit der Oxidationsmittelzubereitung gemäß Tabelle 2 im Gewichtsverhältnis von 4 zu 5 unter Erhalt eines anwendungsbereiten, klaren, gelförmigen Färbemittels vermischt.

Tabelle 2: Oxidationsmittelzusammensetzung

| | |
|---|---|
| Dipicolinsäure | 0,62 |
| Natriumpyrophosphat | 0,03 |
| Turpinal® SL | 1,50 |
| Texapon® NSO | 2,00 |
| Aculyn® 33 A | 12,00 |
| Wasserstoffperoxid (50 %ige wässrige Lösung) | 12,00 |
| Wasser | ad 100 |

Die Ausfärbung erfolgte stets auf Haarsträhnen (Alkinco 6634, natural dark european hair).

Die Haarsträhnen wurden im Rahmen einer Vorbehandlung mit Texapon® NSO Lösung gewaschen, getrocknet und 24 Stunden gelagert. Als Referenzmessung wurde vor der Anwendung der Färbemittel die Kämmarbeit jeder Haarsträhne bestimmt (Siehe Punkt 1.2).

Pro Farbstoffträger E1, E2, V1 sowie V2 wurden jeweils 20 Haarsträhnen angefeuchtet und mit dem Handtuch getrocknet, so dass eine Restfeuchtigkeit im Haar verblieb. Danach wurde je Strähne ein anwendungsbereites klares, gelförmiges Färbemittel in einem Flottenverhältnis von 2 g Anwendungsmischung pro g Haar aufgetragen und über einen Zeitraum von 30 Minuten bei 37°C einwirken gelassen. Anschließend wurden die Strähnen 2 Minuten mit 30°C warmen, fließendem Leitungswasser gespült.

Abschließend wurde erneut die Kämmarbeit jeder Haarsträhne bestimmt (siehe Punkt 1.2).

1.2 Durchführung der Kämmarbeitsmessung

Vor der Arbeitsmessung wurden die Einzelsträhnen ca. 1 Minute in einer Vorkämmapparatur nass gekämmt. Unmittelbar im Anschluss daran wurde von jeder einzelnen Strähne die Arbeit ermittelt, die für 10 Kämmungen aufgewendet werden musste. Die Messergebnisse der 20 Strähnen wurden gemittelt (arithmetisches Mittel = Ø).

Die so erhaltenen gemittelten Messwerte für die Kämmarbeit vor (Referenz) und nach der Färbung wurden verglichen und die Änderung (Erhöhung) der Kämmarbeit auf die Referenzmessung bezogen:

$$\text{Änderung} = (1 - [\text{Ø Kämmarbeit nachher}])/[\text{Ø Kämmarbeit Referenz}]) \cdot 100$$

Die Ergebnisse sind der Tabelle 3 zu entnehmen.

Tabelle 3:

| Rezeptur | Ø Kämmarbeit Referenz (vor der Färbung) [mJ] | Ø Kämmarbeit (nach der Färbung) [mJ] | Änderung der Kämmarbeit [%] |
|---|---|---|---|
| E1 | 61 | 72 | -21 |
| V1 | 51 | 74 | -46 |

Das erfindungsgemäße Mittel E1 bewirkt eine Konditionierung, so dass die Kämmbarkeit lediglich eine Änderung um ca. 20% erfährt.
Nach Anwendung des Mittels V1 ohne die erfindungsgemäße Wirkstoffkombination weisen die Fasern eine deutlich verschlechterte Nasskämmbarkeit (46% Änderung) auf.
Das Haar erfährt durch V1 eine deutlich schlechtere Konditionierung als durch E1.

*2 Trübe Gele*

2.1 Ausfärbung

Es wurde der folgende trübe gelförmige Farbstoffträger nach einem herkömmlichen Verfahren hergestellt (sofern keine anders lautenden Angaben gemacht werden, verstehen sich die Mengenangaben in Gew.-%):

Tabelle 4:

| Rohstoff | Rezeptur 1 |
|---|---|
| Dehydol® LS 2 deo N | 4,9 |
| Eumulgin® CS50 | 5,0 |
| Lorol, techn. | 8,0 |
| Edenor® PK 1805 | 6,8 |
| Texapon® NSO | 4,5 |
| Merquat® 280 | 2,0 |
| Amphoterge® K-2 | 1,0 |
| Erfindungsgemäßes Silikon | 2,0 |
| Monoethanolamin | 5,0 |
| L-Arginin | 1,0 |
| Natriumsulfit | 0,2 |
| Ascorbinsäure | 0,2 |
| Turpinal® SL | 0,2 |
| Parfum | 0,6 |
| p-Toluylendiamin-sulfat | 0,85 |
| 4-Amino-3-methylphenol | 0,50 |
| Resorcin | 0,57 |
| m-Aminophenol | 0,05 |
| 5-Amino-2-methylphenol | 0,15 |
| 2-Amino-6-chlor-4-nitrophenol | 0,07 |
| Wasser | ad 100 |

Diese Gelformulierung wurde mit der in Tabelle 2 beschriebenen Oxidationsmittelzubreitung im Verhältnis 4 : 5 vermischt und in einem Flottenverhältnis von 2 g Anwendungsmischung pro g Haar auf die Haarsträhnen (Alkinco 6634, natural dark european hair) aufgetragen. Nach einer Einwirkungszeit von 30 Minuten bei 37°C wurden die Strähnen 2 Minuten mit 30°C warmen, fließendem Leitungswasser gespült und mit einem Fön getrocknet.

*3 Verzeichnis der eingesetzten Handelsprodukte*

| | |
|---|---|
| Aculyn® 33 A | Acrylpolymer (ca. 28% Festkörper in Wasser; INCI-Bezeichnung: Acrylates Copolymer) |

(fortgesetzt)

| | |
|---|---|
| Amphoterge® K-2 | ca. 39-41% Aktivsubstanzgehalt; INCI-Bezeichnung: Disodium Cocoamphodipropionate (Lonza) |
| Dehydol® LS 2 deo N | $C_{12-14}$-Fettalkohol mit ca. 2-EO-Einheiten (INCI-Bezeichnung: Laureth-2)(Cognis) |
| Edenor® PK 1805 | Ölsäure (INCI-Bezeichnung: Oleic Acid) (Cognis) |
| Erfindungsgemäßes Silikon | Silikon gemäß Beispiel 8 der WO 02/10257 geringfügig modifiziert in der Länge der Silikon- und der Polyetherketter. |
| Eumulgin® CS 50 | Cetylstearylalkohol mit ca. 50 EO-Einheiten (INCI-Bezeichnung: Ceteareth-50) (Cognis) |
| Herbasol Komplex SC | ca. 3,5-6,5% Trockensubstanzgehalt; INCI-Bezeichnung: Propylene Glycol, Aqua (Water), Sorbitol, Mel (Honey Extract), Hamamelis Virginiana (Witch Hazel) Extract, Aesculus Hippocastanum (Horse Chestnut) Seed Extract, Cinnamomum Zeylanicum Bark Extract, Vitis Vinifera (Grape) Leaf Extract, Trifolium Pratense (Clover) Flower Extract, Ribes Nigrum (Black Currant) Leaf Extract, Aspalathus Linearis Leaf Extract, Rosa Gallica Flower Extract, Pterocarpus Santalinus Wood Extract (Cosmetochem) |
| Lorol® techn. | $C_{12-18}$-Fettalkohol (INCI-Bezeichnung: Coconut alcohol) (Cognis) |
| Mergital® CS 50A | Fettalkohol mit ca. 50 EO-Einheiten (INCI-Bezeichnung: Ceteareth-50) (Cognis) |
| Merquat® 280 | Dimethyldiallylammoniumchlorid Acrylsäure Copolymer (ca. 35 Aktivsubstanz in Wasser; INCI-Bezeichnung: Polyquaternium-22) (Ondeo-Nalco) |
| Miranol® C2M SF | ca. 38-40% Festkörper; INCI-Bezeichnung: Disodium Cocoamphodipropionate (Rhodia) |
| Texapon® NSO | Laurylethersulfat, Natriumsalz (ca. 27,5% Aktivsubstanz; INCI-Bezeichnung: Sodium Laureth Sulfate) (Cognis) |
| Turpinal® SL | 1-Hydroxyethan-1,1-diphosphonsäure (ca. 58 - 61% Aktivsubstanzgehalt; INCI-Bezeichnung: Etidronic Acid, Aqua (Water)) (Solutia) |

## Patentansprüche

**1.** Mittel zur Konditionierung keratinischer Fasern, insbesondere menschlicher Haare, enthaltend in einem kosmetisch akzeptablen Träger

- mindestens ein amphoteres Tensid der Formel (I)

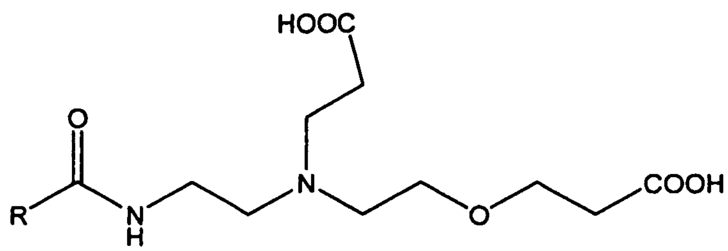

worin R eine lineare oder verzweigte, gesättigte oder ungesättigte ($C_{10}$ - $C_{22}$)-Alkylgruppe bedeutet,

- mindestens ein kationisches und/oder amphoteres Polymer und
- mindestens eine Polyammonium-Polysiloxan-Verbindung.

**2.** Mittel nach Anspruch 1, **dadurch gekennzeichnet, dass** das Polyammonium-Polysiloxan

a1) mindestens eine Polyalkylenoxid-Struktureinheit der allgemeinen Formeln: -A-E-, -E-A-, -A-E-A'- und/oder -A'-E-A- , worin:

A steht für eine der Gruppen: $-CH_2C(O)O-$, $-CH_2CH_2C(O)O-$, $-CH_2CH_2CH_2C(O)O-$, $-OC(O)CH_2-$, $-OC(O)CH_2CH_2-$ und/oder $-OC(O)CH_2CH_2CH_2-$,

A’ bedeutet: $-CH_2C(O)-$, $-CH_2CH_2C(O)-$. $-CH_2CH_2CH_2C(O)-$, $-C(O)CH_2-$, $-C(O)CH_2CH_2-$ und/oder $-C(O)CH_2CH_2CH_2-$ und

E steht für eine Polyalkylenoxidgruppe der allgemeinen Formeln:

$-[CH_2CH_2O]_q-[CH_2CH(CH_3)O]_r-$ und/oder $-[OCH(CH_3)CH_2]_r,-[OCH_2CH_2)_q-$, mit q = 1 bis 200 und r = 0 bis 200, wobei das endständige Sauerstoffatom der Gruppe A an die endständige $-CH_2$-Gruppe der Gruppe E, und das endständige Carbonylkohlenstoffatom der Gruppe A’ an das endständige Sauerstoffatom Gruppe E jeweils unter Ausbildung von Estergruppen binden, und/oder mindestens eine endständige Polyalkylenoxid-Struktureinheit der Formel $-A-E-R^2$, worin A und E die oben genannte Bedeutung aufweisen, und $R^2$ steht für H, geradkettiger, cyclischer oder verzweigter $C_1$ - $C_{20}$ - Kohlenwasserstoffrest, der durch -O-, oder -C(O)unterbrochen und mit -OH substituiert und acetylenisch, olefinisch oder aromatisch sein kann,

a2) mindestens einen zweiwertigen oder dreiwertigen organischen Rest, der mindestens eine Ammoniumgruppe enthält,

a3) mindestens eine Polysiloxan-Struktureinheit der allgemeinen Formel: -K-S-K-,

worin S steht für $-Si(R^1)_2-O[-Si(R^1)_2-O]_nSi(R^1)_2-$ und

worin $R^1$ steht für $C_1$-$C_{22}$-Alkyl, $C_1$-$C_{22}$-Fluoralkyl oder Aryl, n steht für 0 bis 1000, und wenn mehrere Gruppen S in der Polysiloxanverbindung vorliegen, diese gleich oder verschieden sein können,

worin K ein zweiwertiger oder dreiwertiger geradkettiger, cyclischer oder verzweigter $C_2$-$C_{40}$-Kohlenwasserstoffrest, der durch -O-, -N -, $-NR^1-$, -C(O)-, -C(S)-, $-N^+(R^3)-$ und $-N^+(R^1)(R^3)-$ unterbrochen und mit -OH substituiert sein kann,

worin $R^1$ wie oben definiert ist, oder gegebenenfalls eine Bindung zu einem zweiwertigen Rest $R^3$ darstellt, und worin $R^3$ einen einwertigen oder zweiwertigen geradkettigen, cyclischen oder verzweigten $C_1$-$C_{20}$-Kohlenwasserstoffrest, der durch -O-, - NH-, -C(O)-, -C(S)-unterbrochen und mit -OH substituiert sein kann, oder $-A-E-R^2$ darstellt, worin A, E und $R^2$ wie oben definiert ist,

wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt, und

a4) mindestens einen organischen oder anorganischen Säurerest zur Neutralisation der aus der(n) Ammoniumgruppe(n) resultierenden Ladungen

enthält.

3. Mittel nach Anspruch 2, **dadurch gekennzeichnet, dass** das Polyammonium-Polysiloxan eine Verbindung der Formel

$$-[N^4-K-S-K-N^4-A-E-A^-]_m- \text{ bzw. } -[N^4-K-S-K-N^4-A'-E-A]_m- \quad \text{(IV)}$$

ist, wobei K, S, A, E und A’ die oben gegebenen Bedeutungen haben und

$N^4$ ein organischer Rest, der mindestens eine quartäre Ammoniumgruppe enthält, der allgemeinen Formel

$$-(N^+R^{12}R^{13})-$$

ist, worin $R^{12}$ ein einwertiger oder zweiwertiger geradkettiger, cyclischer oder verzweigter $C_1$-$C_{20}$-Kohlenwasserstoffrest ist, der durch -O-, -NH-, -C(O)-, -C(S)- unterbrochen und mit -OH substituiert sein kann,

$R^{13}$ die Bedeutungen von $R^{12}$ aufweisen kann, oder eine Einfachbindung zu K oder $R^{12}$ darstellt, und die Reste $R^{12}$ und $R^{13}$ gleich oder verschieden voneinander sein können.

4. Mittel nach Anspruch 3, **dadurch gekennzeichnet, dass** $R^{12}$ steht für eine $C_1$- bis $C_4$-Alkylgruppe und $R^{13}$ steht für eine Einfachbindung zu K.

5. Mittel nach einem der Ansprüche 2 bis 4, **dadurch gekennzeichnet, dass** K steht für einen Rest

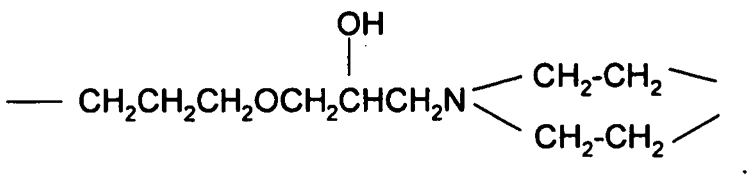

**6.** Mittel nach einem der Ansprüche 2 bis 5, **dadurch gekennzeichnet, dass** S steht für eine Gruppe

$$-Si(R^1)_2-O[-Si(R^1)_2-O]_n-Si(R^1)_2-,$$

worin $R^1$ bedeutet $C_1$-$C_{22}$-Alkyl, $C_1$-$C_{22}$-Fluoralkyl oder Aryl, vorzugsweise Phenyl, n = 0 bis 1000, und wenn mehrere Gruppen S in der Polysiloxanverbindung vorliegen, diese gleich oder verschieden sein können.

**7.** Mittel nach Anspruch 6, **dadurch gekennzeichnet, dass** $R^1$ jeweils für eine Methylgruppe steht.

**8.** Mittel nach einem der Ansprüche 2 bis 7, **dadurch gekennzeichnet, dass** A steht für eine Gruppe $-CH_2CH_2C(O)O-$ A' steht für eine Gruppe $-CH_2CH_2C(O)-$, und E steht für eine Polyalkylengruppe der allgemeinen Formel $-[CH_2CH_2O]_q-$ mit q = 1 bis 200 wobei das endständige Sauerstoffatom der Gruppe A an die endständige $-CH_2$-Gruppe der Gruppe E, und das endständige Carbonylkohlenstoffatom der Gruppe A' an das endständige Sauerstoffatom Gruppe E jeweils unter Ausbildung von Estergruppen binden.

**9.** Mittel nach einem der Ansprüche 1 bis 8 **dadurch gekennzeichnet, dass** die Verbindungen der Formel (I) in einer Menge von 0,1 bis 15 Gew.%, bezogen auf das Gewicht des Mittels, enthalten sind.

**10.** Mittel nach einem der Ansprüche 1 bis 9, **dadurch gekennzeichnet, dass** die amphoteren und/oder kationischen Polymere in einer Menge von 0,1 bis 15 Gew.% bezogen auf das Gewicht des gesamten Mittels, enthalten sind.

**11.** Mittel nach einem der Ansprüche 1 bis 10, **dadurch gekennzeichnet, dass** es weiterhin mindestens ein ($C_8$ bis $C_{22}$)-Triacylglycerid enthält.

**12.** Mittel nach Anspruch 11, **dadurch gekennzeichnet, dass** sich mindestens eine ($C_8$ bis $C_{22}$)-Acylgruppe der ($C_8$ bis $C_{22}$)-Triacylglyceride von mindestens einer ungesättigten Fettsäure ableitet.

**13.** Mittel nach einem der Ansprüche 11 oder 12, **dadurch gekennzeichnet, dass** als ($C_8$ bis $C_{22}$)-Triacylglyceridquelle ein natürliches Öl, insbesondere ein Nussöl, enthalten ist.

**14.** Mittel nach einem der Ansprüche 11 bis 13, **dadurch gekennzeichnet, dass** als ($C_8$ bis $C_{22}$)-Triacylglyceridquelle Macadamianußöl, insbesondere ein Öl aus Macadamia ternifolia, enthalten ist.

**15.** Mittel nach einem der Ansprüche 11 bis 14, **dadurch gekennzeichnet, dass** die ($C_8$ bis $C_{22}$)-Triacylglyceride in einer Menge von 0,05 bis 10 Gew.-%, jeweils bezogen auf das Gewicht des Mittels, enthalten sind.

**16.** Mittel nach einem der Ansprüche 1 bis 15, **dadurch gekennzeichnet, dass** es in Form einer Creme oder eines Gels vorliegt.

**17.** Mittel nach einem der Ansprüche 1 bis 16, **dadurch gekennzeichnet, dass** es zusätzlich eine Kombination aus

- 0,5 bis 10 Gew.-% eines Anlagerungsproduktes von 1 bis 5 Mol Ethylenoxid an einen linearen Fettalkohol mit 8 bis 22 C-Atomen,
- 0,1 bis 15 Gew.% eines gesättigten oder ungesättigten, linearen oder verzweigten Alkohols mit 8 bis 36 C-Atomen und
- 0,1 bis 15 Gew.% einer flüssigen Fettsäure mit 16 bis 22 C-Atomen in Form einer wasserlöslichen Seife

enthält.

**18.** Mittel nach einem der Ansprüche 1 bis 17, **dadurch gekennzeichnet, dass** es zusätzlich 0,5 bis 10 Gew.-% eines

Anlagerungsproduktes von 15 bis 100 Einheiten Ethylenoxid pro Molekül eines linearen Fettalkohols mit 8 bis 22 C-Atomen, enthält.

**19.** Mittel nach einem der Ansprüche 1 bis 18, **dadurch gekennzeichnet, dass** es zusätzlich 3 bis 70 Gew.-% mindestens eines niedermolekularen Monoalkohols und/oder mindestens eines Polyols enthält.

**20.** Mittel nach einem der Ansprüche 1 bis 19, **dadurch gekennzeichnet, dass** es zusätzlich mindestens ein anionisches Acrylsäure- und/oder Methacrylsäure-Polymerisat oder -Copolymerisat enthält.

**21.** Mittel nach einem der Ansprüche 1 bis 20, **dadurch gekennzeichnet, dass** es zusätzlich mindestens ein Pflanzenextrakt enthält.

**22.** Mittel nach einem der Ansprüche 1 bis 17, **dadurch gekennzeichnet, dass** es zusätzlich

- als Oxidationsfarbstoffvorprodukt mindestens eine Entwicklerkomponente und gegebenenfalls mindestens eine Kupplerkomponente
und/oder
- mindestens einen direktziehenden Farbstoff enthält.

**23.** Mittel, welches unmittelbar vor der Anwendung aus zwei Komponenten gemischt wird, **dadurch gekennzeichnet, dass** die erste Komponente ein Mittel gemäß Anspruch 22 bedeutet und die zweite Komponente eine Oxidationsmittelzusammensetzung, enthaltend mindestens ein chemisches Oxidationsmittel, insbesondere Wasserstoffperoxid, ist.

**24.** Verfahren zur Behandlung keratinhaltiger Fasern, bei dem ein Mittel nach einem der Ansprüche 1 bis 23 auf die Fasern aufgetragen wird und nach einer Einwirkzeit wieder abgespült wird.

## Claims

**1.** An agent for conditioning keratinic fibers, in particular human hair, containing in a cosmetically acceptable carrier

- at least one amphoteric surfactant of formula (I)

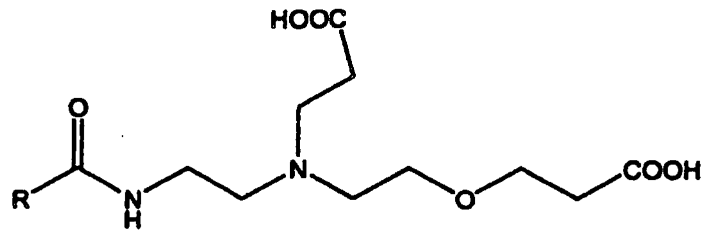

wherein R represents a linear or branched, saturated or unsaturated $C_{10}$-$C_{22}$ alkyl group,

- at least one cationic and/or amphoteric polymer and
- at least one polyammonium-polysiloxane compound.

**2.** The agent according to claim 1, **characterized in that**, the polyammonium-polysiloxane contains

a1) at least one polyakylene oxide structural unit of general formula: -A-E-, -E-A, -A-E-A'- and/or A'-E-A-, wherein:

A represents one of the groups: $-CH_2C(O)O-$, $-CH_2CH_2C(O)O-$, $-CH_2CH_2CH_2C(O)O-$, $-OC(O)CH_2-$, $-OC(O)CH_2CH_2-$, and/or $-OC(O)CH_2CH_2CH_2-$,
A' represents: $-CH_2C(O)-$, $-CH_2CH_2C(O)-$, $-CH_2CH_2CH_2C(O)-$, $-C(O)CH_2-$, $-C(O)CH_2CH_2-$, and/or $-C(O)CH_2CH_2CH_2-$ and
E represents a polyalkylene oxide group of general formulae:

$$-[CH_2CH_2\text{-}O]_q\text{-}[CH_2CH(CH_3)O]_r\text{- and/or -}[OCH(CH_3)CH_2]_r[OCH_2CH_2]_q\text{-},$$

with q = 1 to 200 and r = 0 to 200, wherein the terminal oxygen atom of group A binds to the terminal $-CH_2$ group of group E, and the terminal carbonyl carbon atom of group A' binds to the terminal oxygen atom of group E respectively, with formation of ester groups, and/or at least one terminal polyalkylene oxide structural unit of formula $-A-E-R^2$, wherein A and E have the meaning mentioned above, and $R^2$ represents H, a linear, cyclic or branched $C_1$-$C_{20}$ hydrocarbon radical, which may be interrupted by -O- or -C(O) and substituted with -OH, and may be acetylenic, olefinic or aromatic,

a2) at least one bivalent or trivalent organic radical which contains at least one ammonium group,
a3) at least one polysiloxane structural unit of general formula -K-S-K-,
wherein S represents $-Si(R^1)_2-O[-Si(R^1)_2-O]_n-Si(R^1)_2-$ and
wherein $R^1$ represents a $C_1$-$C_{22}$ alkyl, $C_1$-$C_{22}$ fluoroalkyl or aryl, n represents 0 to 1,000, and if several groups S exist in the polysiloxane compound, these may be identical or different,
wherein K represents a bivalent or trivalent, linear, cyclic or branched $C_2$-$C_{40}$ hydrocarbon radical, which may be interrupted by -O-, -N-, $-NR^1-$, -C(O)-, -C(S)-, $-N^+(R^3)$ and $-N^+(R^1)(R^3)-$ and substituted with -OH,
wherein $R^1$ is defined as above, or optionally represents a bond to a bivalent radical $R^3$, and
wherein $R^3$ represents a monovalent or bivalent, linear, cyclic, or branched $C_1$-$C_{20}$ hydrocarbon radical, which may be interrupted by -O-, - NH-, -C(O)-, -C(S)- and substituted with -OH, or $-A-E-R^2$, wherein A, E and $R^2$ are defined as above,
wherein the radicals K may be identical or different from each other, and in the case, when K represents a trivalent radical, the saturation of the third valence via a bond to the organic radical mentioned earlier, which contains at least one ammonium group, and
a4) at least one organic or inorganic acid radical for neutralizing the charges resulting from the ammonium group(s).

3. The agent according to claim 2, **characterized in that** the polyammonium-polysiloxane is a compound of formula

$$-[N^4\text{-K-S-K-}N^4\text{-A-E-A'}]_m\text{- or -}[N^4\text{-K-S-K-}N^4\text{-A'-E-A}]_m\text{-} \quad \text{(IV)}$$

wherein K, S, A, E and A' have the meanings indicated above and
$N^4$ is an organic radical, which contains at least one quaternary ammonium group, of general formula

$$-(N^+R^{12}R^{13})-$$

wherein $R^{12}$ is a monovalent or bivalent, linear, cyclic or branched $C_1$-$C_{20}$ hydrocarbon radical, which may be interrupted by -O-, -NH-, -C(O)-, -C(S)- and substituted with -OH,
$R^{13}$ may have the meanings of $R^{12}$, or represents a single bond to K or to $R^{12}$, and the radicals $R^{12}$ and $R^{13}$ may be identical or different from each other.

4. The agent according to claim 3, **characterized in that** $R^{12}$ represents a $C_1$-$C_4$ alkyl group and $R^{13}$ represents a single bond to K.

5. The agent according to any of claims 2 to 4, **characterized in that** K represents a radical

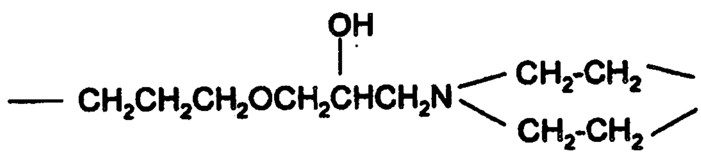

6. The agent according to any of claims 2 to 5, **characterized in that** S represents a group

$$-Si(R^1)_2-O[-Si(R^1)_2-O]_n-Si(R^1)_2-,$$

wherein $R^1$ represents a $C_1$-$C_{22}$ alkyl, $C_1$-$C_{22}$ fluoroalkyl or aryl, preferably phenyl, n = 0 to 1,000, and if several

groups S exist in the polysiloxane compound, these may be identical or different.

**7.** The agent according to claim 6, **characterized in that** $R^1$ each represents a methyl group.

**8.** The agent according to any of claims 2 to 7, **characterized in that** A represents a $-CH_2CH_2C(O)O-$ group, A' represents a $-CH_2CH_2C(O)-$ group, and E represents a polyalkylene group of general formula $-[CH_2CH_2-O]_q-$ with q = 1 to 200, wherein the terminal oxygen atom of group A binds to the terminal $-CH_2$ group of group E, and the terminal carbonyl carbon atom of group A' binds to the terminal oxygen atom of group E, respectively with formation of ester groups.

**9.** The agent according to any of claims 1 to 8, **characterized in that** the compounds of formula (I) are contained in an amount from 0.1 to 15% by weight, based on the weight of the agent.

**10.** The agent according to any of claims 1 to 9, **characterized in that** the amphoteric and/or cationic polymers are contained in an amount from 0.1 to 15% by weight, based on the weight of the whole agent.

**11.** The agent according to any of claims 1 to 10, **characterized in that** it further contains at least one $C_8$-$C_{22}$ triacylglyceride.

**12.** The agent according to claim 11, **characterized in that** at least one $C_8$-$C_{22}$ acyl group of the $C_8$-$C_{22}$ triacylglycerides is derived from at least one unsaturated fatty acid.

**13.** The agent according to any of claims 11 or 12, **characterized in that** a natural oil, in particular a nut oil is contained as a $C_8$-$C_{22}$ triacylglyceride source.

**14.** The agent according to any of claims 11 to 13, **characterized in that** macadamia nut oil, in particular an oil from *Macadamia ternifolia* is contained as a $C_8$-$C_{22}$ triacylglyceride source.

**15.** The agent according to any of claims 11 to 14, **characterized in that** the $C_8$-$C_{22}$ triacylglycerides are contained in an amount from 0.05 to 10% by weight, each based on the weight of the agent.

**16.** The agent according to any of claims 1 to 15, **characterized in that** it exists as a cream or a gel.

**17.** The agent according to any of claims 1 to 16, **characterized in that** it additionally contains a combination of

- 0.5 to 10% by weight of an addition product of 1 to 5 moles of ethylene oxide on a linear fatty alcohol with 8 to 22 C atoms,
- 0.1 to 15% by weight of a saturated or unsaturated, linear or branched alcohol with 8 to 36 C atoms and
- 0.1 to 15% by weight of a liquid fatty acid with 16 to 22 C atoms as a water-soluble soap.

**18.** The agent according to any of claims 1 to 17, **characterized in that** it additionally contains 0.5 to 10% by weight of an addition product of 15 to 100 ethylene oxide units per molecule of a linear fatty alcohol with 8 to 22 C atoms.

**19.** The agent according to any of claims 1 to 18, **characterized in that** it additionally contains 3 to 70% by weight of at least one low molecular weight mono-alcohol and/or at least one polyol.

**20.** The agent according to any of claims 1 to 18, **characterized in that** it additionally contains at least one anionic acrylic acid and/or methacrylic acid polymer or copolymer.

**21.** The agent according to any of claims 1 to 20, **characterized in that** it additionally contains at least one plant extract.

**22.** The agent according to any of claims 1 to 17, **characterized in that** it additionally contains

- at least one developer component and optionally at least one coupling agent as oxidation dye precursor product and/or
- at least one substantive dye.

**23.** An agent, which is mixed directly out of two components before application, **characterized in that** the first component

represents an agent according to claim 22 and the second component is an oxidizer composition, containing at least one chemical oxidizer, in particular hydrogen peroxide.

**24.** A method for treating keratinic fibers, wherein an agent according to any of claims 1 to 23 is applied on the fibers and is rinsed again after an action time.

**Revendications**

**1.** Agent pour le conditionnement de fibres kératiniques, en particulier de cheveux humains, contenant, dans un support acceptable du point de vue cosmétique

- au moins un agent tensioactif amphotère répondant à la formule (I)

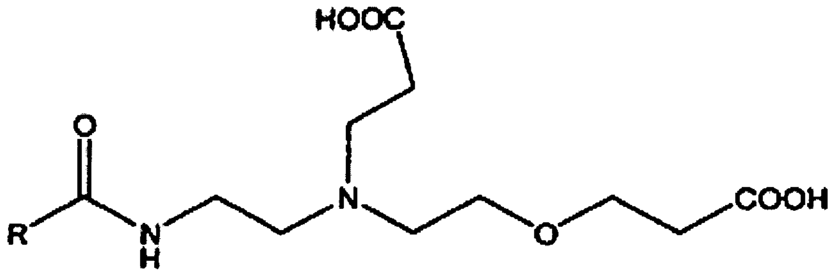

dans laquelle R représente un groupe alkyle en $C_{10}$-$C_{22}$ à chaîne droite ou ramifiée, saturé ou insaturé,

- au moins un polymère cationique et/ou amphotère ; et
- au moins un composé de polyammonium-polysiloxane.

**2.** Agent selon la revendication 1, **caractérisé en ce que** le polyammonium-polysiloxane contient :

a1) au moins une unité de structure d'oxyde de polyalkylène répondant aux formules générales : -A-E-, -E-A-, -A-E-A'- et/ou -A'-E-A-, dans lesquelles :

A représente un des groupes : $-CH_2C(O)O-$, $-CH_2CH_2C(O)O-$, $-CH_2CH_2CH_2C(O)O-$, $-OC(O)CH_2-$, $-OC(O)CH_2CH_2-$ et/ou $-OC(O)CH_2CH_2CH_2-$,
A' représente un groupe $-CH_2C(O)-$, un groupe $-CH_2CH_2C(O)-$, un groupe $-CH_2CH_2CH_2C(O)-$, un groupe $-C(O)CH_2-$, un groupe - $C(O)CH_2CH_2-$ et/ou un groupe $-C(O)CH_2CH_2CH_2-$ et
E représente un groupe d'oxyde de polyalkylène répondant aux formules générales :

$-[CH_2CH_2O]_q-[CH_2CH(CH_3)O]_r-$ et/ou $-[OCH(CH_3)CH_2]_r$ $-[OCH_2CH_2]_q$ , où q = 1 à 200 et r = 0 à 200, l'atome d'oxygène terminal du groupe A sur le groupe terminal $-CH_2-$ du groupe E et l'atome de carbone terminal du groupe carbonyle du groupe A' sur l'atome d'oxygène terminal du groupe E se liant respectivement en formant des groupes esters, et/ou au moins une unité de structure terminale d'oxyde de polyalkylène répondant à la formule $-A-E-R^2$, dans laquelle A et E présentent la signification mentionnée ci-dessus, et $R^2$ représente un atome d'hydrogène, un radical d'hydrocarbure en $C_1$-$C_{20}$ à chaîne droite, cyclique ou ramifié, qui peut être interrompu par un atome d'oxygène ou par un groupe - C(O)- et qui peut être substitué avec un groupe -OH et qui peut être de type acétylénique, oléfinique ou aromatique ;

a2) au moins un radical organique bivalent ou trivalent, qui contient au moins un groupe ammonium ;
a3) au moins une unité de structure de polysiloxane répondant à la formule générale -K-S-K-,
dans laquelle S représente un groupe $-Si(R^1)_2-O[-Si(R^1)_2-O]_n-Si(R^1)_2-$ et
dans laquelle $R^1$ représente un groupe alkyle en $C_1$-$C_{22}$, un groupe fluoroalkyle en $C_1$-$C_{22}$ ou un groupe aryle, n est égal à 0-1000, et lorsque plusieurs groupes S sont présents dans le composé de polysiloxane, ces groupes peuvent être identiques ou différents,
dans laquelle K représente un radical d'hydrocarbure en $C_2$-$C_{40}$ bivalent ou trivalent à chaîne droite, cyclique

ou ramifié, qui peut être interrompu par un atome d'oxygène, un atome d'azote, un groupe -$NR^1$-, un groupe -C(O)-, un groupe -C(S)-, un groupe - $N^+(R^3)$- et un groupe -$N^+(R^1)(R^3)$- et qui peut être substitué avec un groupe -OH,
$R^1$ étant tel que défini ci-dessus ou représentant le cas échéant une liaison à un radical bivalent $R^3$, et
$R^3$ représentant un radical d'hydrocarbure en $C_1$-$C_{20}$ monovalent ou bivalent à chaîne droite, cyclique ou ramifié qui peut être interrompu par un atome d'oxygène, un groupe -NH-, un groupe - C(O)-, un groupe -C(S)-, et qui peut être substitué avec un groupe -OH, ou bien représentant un groupe -A-E-$R^2$ dans lequel A, E et $R^2$ sont tels que définis ci-dessus,
les radicaux K pouvant être identiques ou différents l'un de l'autre et, dans le cas où K représente un radical trivalent, la saturation de la troisième valence a lieu via une liaison au radical organique mentionné ci-dessus qui contient au moins un groupe ammonium, et
a4) au moins un radical d'acide organique ou inorganique pour la neutralisation des charges résultant du/des groupes ammonium.

3. Agent selon la revendication 2, **caractérisé en ce que** le polyammonium-polysiloxane représente un composé répondant à la formule

$$-[N^4\text{-K-S-K-}N^4\text{-A-E-A'}]_m \text{ resp. } -[N^4\text{-K-S-K-}N^4\text{-A'-E-A}]_m \qquad \text{(IV)}$$

dans laquelle K, S, A, E et A' ont les significations indiquées ci-dessus et $N^4$ représente un radical organique qui contient au moins un groupe d'ammonium quaternaire, répondant à la formule générale

$$-(N^+R^{12}R^{13})-$$

$R^{12}$ représentant un radical d'hydrocarbure en $C_1$-$C_{20}$ monovalent ou bivalent à chaîne droite, cyclique ou ramifié, qui peut être interrompu par un atome d'oxygène, par un groupe -NH-, par un groupe -C(O)-, par un groupe -C(S)-, et qui peut être substitué avec un groupe -OH,
$R^{13}$ pouvant présenter les significations de $R^{12}$ ou représentant une liaison simple à K ou à $R^{12}$, et les radicaux $R^{12}$ et $R^{13}$ pouvant être identiques ou différents l'un de l'autre.

4. Agent selon la revendication 3, **caractérisé en ce que** $R^{12}$ représente un groupe alkyle en $C_1$-$C_4$, et $R^{13}$ représente une liaison simple à K.

5. Agent selon l'une quelconque des revendications 2 à 4, **caractérisé en ce que** K représente un radical

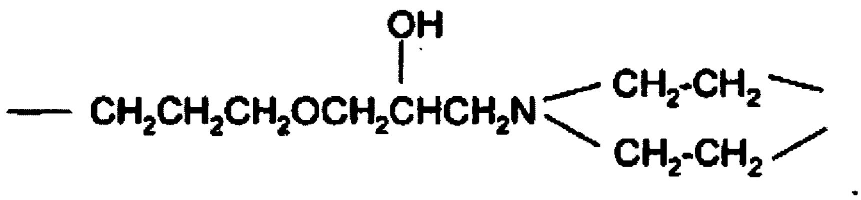

6. Agent selon l'une quelconque des revendications 2 à 5, **caractérisé en ce que** S représente un groupe

$$-Si(R^1)_2\text{-O}[-Si(R^1)_2\text{-O}]_n\text{- }Si(R^1)_2\text{-},$$

dans lequel $R^1$ représente un groupe alkyle en $C_1$-$C_{22}$, un groupe fluoroalkyle en $C_1$-$C_{22}$ ou un groupe aryle, de préférence un groupe phényle, n est égal à 0-1000, et lorsque plusieurs groupes S sont présents dans le composé de polysiloxane, ces groupes peuvent être identiques ou différents.

7. Agent selon la revendication 6, **caractérisé en ce que** $R^1$ représente respectivement un groupe méthyle.

8. Agent selon l'une quelconque des revendications 2 à 7, **caractérisé en ce que** A représente un groupe -$CH_2CH_2C$(O)O-, A' représente un groupe - $CH_2CH_2C$(O)-, et E représente un groupe polyalkylène répondant à la formule générale -$[CH_2CH_2O]_q$ dans laquelle q = 1 à 200, l'atome d'oxygène terminal du groupe A sur le groupe terminal -$CH_2$- du groupe E et l'atome de carbone terminal du groupe carbonyle du groupe A' sur l'atome d'oxygène terminal

du groupe E se liant respectivement en formant des groupes esters.

**9.** Agent selon l'une quelconque des revendications 1 à 8, **caractérisé en ce que** les composés répondant à la formule (I) sont contenus en une quantité de 0,1 à 15 % en poids, rapportés au poids de l'agent.

**10.** Agent selon l'une quelconque des revendications 1 à 9, **caractérisé en ce que** les polymères amphotères et/ou cationiques sont contenus en une quantité de 0,1 à 15 % en poids, rapportés au poids de l'agent total.

**11.** Agent selon l'une quelconque des revendications 1 à 10, **caractérisé en ce qu'**il contient en outre au moins un triacylglycéride en $C_8$-$C_{22}$.

**12.** Agent selon la revendication 11, **caractérisé en ce qu'**au moins un groupe acyle en $C_8$-$C_{22}$ des triacylglycérides en $C_8$-$C_{22}$ dérive d'au moins un acide gras insaturé.

**13.** Agent selon l'une quelconque des revendications 11 ou 12, **caractérisé en ce qu'**il contient, à titre de source de triacylglycéride en $C_8$-$C_{22}$, une huile naturelle, en particulier une huile de noix.

**14.** Agent selon l'une quelconque des revendications 11 à 13, **caractérisé en ce qu'**il contient, à titre de source de triacylglycéride en $C_8$-$C_{22}$, de l'huile de noix de macadamia, en particulier une huile de Macadamia ternifolia.

**15.** Agent selon l'une quelconque des revendications 1 à 14, **caractérisé en ce qu'**il contient les triacylglycérides en $C_8$-$C_{22}$ en une quantité de 0,05 à 10 % en poids, chaque fois rapportés au poids de l'agent.

**16.** Agent selon l'une quelconque des revendications 1 à 15, **caractérisé en ce qu'**il est présent sous la forme d'une crème ou d'un gel.

**17.** Agent selon l'une quelconque des revendications 1 à 16, **caractérisé en ce qu'**il contient en outre une combinaison de :

- à concurrence de 0,5 à 10 % en poids, un produit d'addition de 1 à 5 mol d'oxyde d'éthylène à un alcool gras linéaire contenant de 8 à 22 atomes de carbone ;
- à concurrence de 0,1 à 15 % en poids, un alcool linéaire ou ramifié, saturé ou insaturé, contenant de 8 à 36 atomes de carbone ; et
- à concurrence de 0,1 à 15 % en poids, un acide gras liquide contenant de 16 à 22 atomes de carbone, sous la forme d'un savon soluble dans l'eau.

**18.** Agent selon l'une quelconque des revendications 1 à 17, **caractérisé en ce qu'**il contient en outre, à concurrence de 0,5 à 10 % en poids, un produit d'addition de 15 à 100 unités d'oxyde d'éthylène par molécule d'un alcool gras linéaire contenant de 8 à 22 atomes de carbone.

**19.** Agent selon l'une quelconque des revendications 1 à 18, **caractérisé en ce qu'**il contient en outre, à concurrence de 3 à 70 % en poids, au moins un alcool monovalent à bas poids moléculaire et/ou au moins un polyol.

**20.** Agent selon l'une quelconque des revendications 1 à 18, **caractérisé en ce qu'**il contient en outre au moins un polymère ou un copolymère anionique d'acide acrylique et/ou d'acide méthacrylique.

**21.** Agent selon l'une quelconque des revendications 1 à 20, **caractérisé en ce qu'**il contient en outre au moins un extrait végétal.

**22.** Agent selon l'une quelconque des revendications 1 à 17, **caractérisé en ce qu'**il contient en outre

- à titre de précurseur de colorant d'oxydation, au moins un composant faisant office de développeur et, le cas échéant, au moins un composant faisant office de coupleur
et/ou
- au moins un colorant direct.

**23.** Agent, qui est mélangé directement avant l'application à partir de deux composants, **caractérisé en ce que** le premier composant désigne un agent selon la revendication 22 et le deuxième composant représente une composition d'agents d'oxydation contenant au moins un agent d'oxydation chimique, en particulier du peroxyde d'hydrogène.

**24.** Procédé pour le traitement de fibres kératiniques, dans lequel on applique sur les fibres un agent selon l'une quelconque des revendications 1 à 23 et, après l'avoir laissé agir pendant un certain temps, on l'en élimine à nouveau par rinçage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4507280 A
- EP 640335 B1
- WO 2004069137 A
- WO 0210257 A
- DE 10304923 A1, GE BAYER SILICONES GMBH & CO [DE]
- GB 2104091 A
- EP 47714 A
- EP 217274 A
- EP 283817 A
- DE 2817369
- DE 3929973
- FR 2252840 A
- DE 1165574 C
- DE 2024051 C
- US 3101300 A
- US 3101301 A
- DE 3843713 A1
- DE 3504242
- GB 870994 A
- DE 1164095
- DE 3445549
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- EP 998908 A2


### In der Beschreibung aufgeführte Nicht-Patentliteratur


- *Faraday discussions of the chemical society,* 1974, (57), 7-18
- **HAPPI.** *Household Pers. Prod. Ind.,* 1993, vol. 30 (2), 58-64
- *Angewandte Chemie,* 1952, vol. 64, 213-20
- **KH. SCHRADER.** Grundlagen und Rezepturen der Kosmetika. Hüthig Buch Verlag, 1989